# EUROPÄISCHE PATENTSCHRIFT

(11) **EP 1 989 198 B1**
(45) Veröffentlichungstag und Bekanntmachung des Hinweises auf die Patenterteilung: **09.10.2013**
(21) Anmeldenummer: 07704662.1
(22) Anmeldetag: 20.02.2007
(51) Int. Cl.: C07D 403/12, C07D 413/12, C07D 498/04, A61K 31/551, A61K 31/553, A61P 29/00, A61P 35/00, A61P 37/00

(54) **2, 4-DIAMINOPYRIMIDINDERIVATE ZUR BEHANDLUNG UND/ODER PRÄVENTION VON KREBS, INFEKTIONEN, ENTZÜNDUNGS- UND AUTOIMMUNERKRANKUNGEN**
2,4-DIAMINOPYRIMIDINE DERIVATIVES FOR THE TREATMENT AND/OR PREVENTION OF CANCER, INFECTIONS, INFLAMMATORY AND AUTOIMMUNE DISORDERS
DÉRIVÉS DE 2,4-DIAMINOPYRIMIDINE POUR LE TRAITEMENT ET/OU LA PRÉVENTION DE MALADIES CANCÉREUSES, INFECTIEUSES, INFLAMMATOIRES ET AUTOIMMUNES

(30) Priorität: 22.02.2006 EP 06110303
(43) Veröffentlichungstag der Anmeldung: 12.11.2008
(73) Patentinhaber: Boehringer Ingelheim International GmbH, 55216 Ingelheim am Rhein (DE)
(72) Erfinder: STADTMUELLER, Heinz, A-1120 Vienna (AT); BAUM, Anke, A-1050 Vienna (AT); BOEHMELT, Guido, A-2531 Gaaden (AT); ENGELHARDT, Harald, A-2483 Ebreichsdorf (AT); QUANT, Jens, A-2380 Perchtoldsdorf (AT); SOLCA, Flavio, A-1230 Wien (AT); STEEGMAIER, Martin, 72762 Reutlingen (DE); ZAHN, Stephan Karl, A-1130 Vienna (AT)
(74) Vertreter: Simon, Elke Anna Maria
(86) Internationale Anmeldenummer: PCT/EP2007/051599
(87) Internationale Veröffentlichungsnummer: WO 2007/096351

(56) Entgegenhaltungen:
- WO-A-2004/074244
- WO-A-2004/080980
- WO-A-2006/021544

## Beschreibung

Die vorliegende Erfindung betrifft neue 2,4-Diaminopyrimidine der allgemeinen Formel (1) 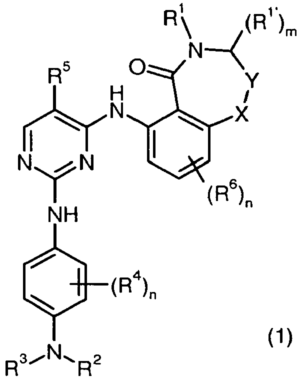 wobei die Reste R¹ bis R⁶, X und Y die in den Ansprüchen und der Beschreibung genannten Bedeutungen haben, deren Isomere, Verfahren zur Herstellung dieser Pyrimidine sowie deren Verwendung als Arzneimittel.

### Hintergrund der Erfindung

Tumorzellen entziehen sich teilweise oder völlig der Regulation und Kontrolle durch den Organismus und zeichnen sich durch ein unkontrolliertes Wachstum aus. Dies beruht einerseits auf dem Verlust von Kontrollproteinen, wie z.B. Rb, p16, p21 und p53 als auch auf der Aktivierung von so genannten Beschleunigern des Zellzykluses, den cyclin-abhängigen Kinasen (CDK's).

Studien in Modellorganismen wie *Schizosaccharomyces pombe, Drosophila melanogaster* oder *Xenopus laevis* sowie Untersuchungen in menschlichen Zellen haben gezeigt, dass der Übergang von der G2 Phase zur Mitose durch die CDK1/Cyclin B Kinase reguliert wird (Nurse 1990, Nature 344: 503-508). Diese Kinase, die auch als "mitosis promoting factor" (MPF) bezeichnet wird, phosphoryliert und reguliert dadurch eine Vielzahl von Proteinen, wie z.B. nukleäre Lamine, kinesinähnliche Motorproteine, Kondensine und Golgi Matrix Proteine, die eine wichtige Rolle beim Abbau der Kernhülle, bei der Zentrosomen Separation, dem Aufbau des mitotischen Spindelapparates, der Chromosomen Kondensation und Abbau des Golgi Apparates spielen (Nigg. E. 2001, Nat Rev Mol Cell Biol. 2(1):21-32). Eine murine Zelllinie mit einer temperatursensitiven CDK1 Kinasemutante zeigt nach Temperaturerhöhung einen raschen Abbau der CDK1 Kinase und einen darauf folgenden Arrest in der G2/M Phase (Th'ng et al. 1990, Cell. 63(2):313-24). Die Behandlung von humanen Tumorzellen mit Inhibitoren gegen CDK1/Cyclin B, wie z.B. Butyrolacton, führt zu einem Arrest in der G2/M Phase und anschließender Apoptose (Nishio, et al. 1996, Anticancer Res.16(6B):3387-95).

Darüber hinaus wird auch für die Proteinkinase Aurora B eine essentielle Funktion beim Eintritt in die Mitose beschrieben. Aurora B phosphoryliert Histon H3 an Ser10 und leitet damit die Chromosomenkondensation ein (Hsu et al. 2000, Cell 102:279-91). Ein spezifischer Zellzyklusarrest in der G2/M Phase kann aber auch z.B. durch Inhibition von spezifischen Phosphatasen wie z.B. Cdc25C (Russell and Nurse 1986, Cell 45:145-53) ausgelöst werden. Hefen mit defektem Cdc25 Gen arretieren in der G2 Phase, während eine Überexpression von Cdc25 zu einem verfrühten Eintritt in die Mitosephase führt (Russell und Nurse 1987, Cell 49:559-67). Des weiteren kann ein Arrest in der G2/M Phase auch durch Inhibition von bestimmten Motorproteinen, den so genannten Kinesinen wie z.B. Eg5 (Mayer et al. 1999, Science 286:971-4), oder durch Mikrotubuli stabilisierende oder destabilisierende Agentien (z.B. Colchicin, Taxol, Etoposid, Vinblastin, Vincristin) ausgelöst werden (Schiff und Horwitz 1980, Proc Natl Acad Sci U S A 77:1561-5).

Neben den Cyclin-abhängigen und den Aurora Kinasen spielen des weiteren die so genannten Polo-like Kinasen, eine kleine Familie von Serin/Threonin-Kinasen, eine wichtige Rolle bei der Regulation des eukaryontischen Zellzykluses. Bisher wurden die Polo-like Kinasen PLK-1, PLK-2, PLK-3 und PLK-4 in der Literatur beschrieben. Besonders für PLK-1 wurde eine zentrale Rolle in der Regulation der Mitosephase gezeigt. PLK-1 ist für die Reifung der Zentrosomen, für die Aktivierung der Phosphatase Cdc25C, sowie für die Aktivierung des Anaphase Promoting Complex verantwortlich (Glover et al. 1998, Genes Dev. 12:3777-87; Qian et al. 2001, Mol Biol Cell. 12:1791-9). Die Injektion von PLK-1 Antikörpern führt zu einem G2 Arrest in nicht transformierten Zellen, während Tumorzellen in der Mitosephase arretieren (Lane und Nigg 1996, J Cell Biol. 135:1701-13). Überexpression von PLK-1 konnte für verschiedene Tumorarten, wie nicht kleinzelliges Lungenkarzinom, Plattenepithelkarzinom, Brust- und kolorektales Karzinom (Wolf et al. 1997, Oncogene 14:543-549; Knecht et al. 1999, Cancer Res. 59:2794-2797; Wolf et al. 2000, Pathol. Res. Pract. 196:753-759; Takahashi et al. 2003, Cancer Sci. 94:148-52) gezeigt werden. Daher stellt diese Klasse von Proteinen ebenfalls einen interessanten Angriffspunkt zur therapeutischen Intervention proliferativer Krankheiten dar (Liu and Erikson 2003, Proc Natl Acad Sci U S A 100:5789-5794).

Die Resistenz vieler Tumorarten erfordert die Entwicklung neuer Arzneimittel zur Tumorbekämpfung. Der vorliegenden Erfindung liegt daher die Aufgabe zugrunde, neue Wirkstoffe aufzuzeigen, welche zur Vorbeugung und/oder Behandlung von Krankheiten, die durch exzessive oder anomale Zellproliferation charakterisiert sind, eingesetzt werden können.

### Detaillierte Beschreibung der Erfindung

Es wurde nun überraschenderweise gefunden, dass Verbindungen der allgemeinen Formel (1), worin die Reste R¹ bis R⁶, X und Y die nachstehend genannten Bedeutungen haben, als Inhibitoren spezifischer Zellzykluskinasen wirken. Somit können die erfindungsgemäßen Verbindungen beispielsweise zur Behandlung von Erkrankungen, die mit der Aktivität spezifischer Zellzykluskinasen in Zusammenhang stehen und durch exzessive oder anomale Zellproliferation charakterisiert sind, verwendet werden.

Die vorliegende Erfindung betrifft Verbindungen der allgemeinen Formel (1) 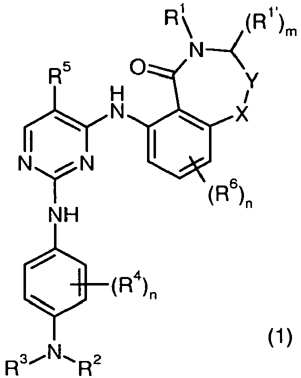 worin
X Sauerstoff oder Stickstoff, und
Y -CH₂- oder C=O, und
R¹ und R^{1'} jeweils unabhängig voneinander Wasserstoff oder ein gegebenenfalls einfach oder mehrfach substituierter Rest ausgewählt aus der Gruppe bestehend aus C₁₅Alkyl, C₃₋₆Cycloalkyl, Aryl, Heterocyclyl und Heteroaryl, wobei der/die Substituenten gleich oder verschieden sein können und ausgewählt werden aus der Gruppe bestehend aus Halogen, -OR^{a}, -C(=O)R^{a}, -C(=O)OR^{a}, -C(=O)NR^{a}R^{a}, -NR^{a}R^{a}, -NR^{a}C(=O)R^{a}, -NR^{a}C(=O)OR^{a}, -NR^{a}C(=O)NR^{a}R^{a}, -NR^{a}SO₂R^{a}, -N=CR^{a}R^{a}, -SR^{a}, -SOR^{a}, -SO₂R³, -SO₂NR^{a}R^{a}, -NR-SO₂NR^{a}R^{a}, -OSO₂NR^{a}R^{a} und Pseudohalogen, oder gegebenenfalls können die am gleichen C-Atom befindlichen R^{1'} miteinander einen C₃₋₇Cycloalkylring bilden,
oder gegebenenfalls kann R¹ mit einem R^{1'} eine gemeinsame gesättigte oder teilweise ungesättigte 3-6-gliedrigen Alkylbrücke, die gegebenenfalls ein bis zwei Heteroatome enthalten kann, verbunden sein, und
R² ein gegebenenfalls einfach oder mehrfach substituierter Heterocyclyl Rest, wobei der/die Substituenten gleich oder verschieden sein können und ausgewählt werden aus der Gruppe bestehend aus C₁₋₅Alkyl, C₂₋₅Alkenyl, C₂₋₅Alkinyl, C₃₋₆CycloalkylC₀₋₃Alkyl, und
R³ Wasserstoff oder C₁₋₃Alkyl, oder
R² und R³ gemeinsam einen gegebenenfalls einfach oder mehrfach substituierten Heterocyclylring bilden, der gegebenenfalls ein bis zwei weitere Heteroatome enthalten kann, wobei der/die Substituenten gleich oder verschieden sein können und ausgewählt werden aus der Gruppe bestehend aus C₁₋₅Alkyl, C₂₋₅Alkenyl, C₂₋₅Alkinyl und C₃₋₆CycloalkylC₀₋₃Alkyl, oder ein geeigneter Substituent ausgewählt aus der Gruppe bestehend aus Halogen, -OR³, -C(=O)R^{a}, -C(=O)OR^{a}, -C(=O)NR^{a}R^{a}, -NR^{a}R^{a}, - NR^{a}C(=O)R^{a}, -NR^{a}C(=O)OR^{a}, -NR^{a}C(=O)NR^{a}R^{a}, -NR^{a}SO₂R^{a}, -N=CR^{a}R^{a}, -SR^{a}, -SOR^{a}, -SO₂R^{a}, -SO₂NR^{a}R^{a}, -NR^{a}SO₂NR^{a}R^{a}, -OSO₂NR^{a}R^{a} und Pseudohalogen, und
R⁴ jeweils unabhängig voneinander ein Rest ausgewählt aus der Gruppe bestehend aus Halogen, NO₂, -OR^{a}, -C(=O)R^{a}, -C(=O)OR^{a}, -C(=O)NR^{a}R^{a}, -NR^{a}R^{a}, -NR^{a}COR^{a}, -NR^{a}C(=O)OR^{a}, -NR^{a}C(=O)NR^{a}R^{a}, -NR^{a}C(=O)ONR^{a}R^{a}, -NR^{a}SO₂R^{a}, -N=CR^{a}R^{a}, -SR^{a}, -SOR^{a}, -SO₂R^{a}, -SO₂NR^{a}R^{a}, -NR^{a}SO₂NR^{a}R^{a}, -OSO₂NR^{a}R^{a} und Pseudohalogen; und
R⁵ Wasserstoff, Halogen, C₁₋₃Alkyl, C₂₋₃Alkenyl, C₂₋₃Alkinyl, Halogen-C₁₋₃Alkyl-, -OR^{a} oder Pseudohalogen, und
R⁶ jeweils unabhängig voneinander ein Rest ausgewählt aus der Gruppe bestehend aus Halogen, C₁₋₃Alkyl, -OR^{a} und Pseudohalogen, und
R^{a} jeweils unabhängig voneinander Wasserstoff oder ein Rest ausgewählt aus der Gruppe bestehend aus gegebenenfalls substituiertem C₁₋₈Alkyl, C₂₋₈Alkenyl, C₂₋₈Alkinyl, C₃₋₁₀Cycloalkyl, Aryl, Heterocyclyl und Heteroaryl, wobei der/die Substituent(en) gleich oder verschieden sein können und ausgewählt werden aus der Gruppe bestehend aus Halogen, -NH₂, -OH und Pseudohalogen; und
m 0, 1 oder 2; und
n jeweils unabhängig voneinander 0, 1 oder 2
bedeuten, gegebenenfalls in Form ihrer Tautomeren, ihrer Racemate, ihrer Enantiomere, ihrer Diastereomere und ihrer Gemische, sowie gegebenenfalls ihrer pharmakologisch verträglichen Salze, mit der Maßgabe, dass
2-(2-Methoxy-4-piperazin-1-yl-phenylamino)-4-(3,3-dimethyl-5-oxo-2,3,4,5-benzo[*f*][1,4]oxazepin-6-ylamino-5-trifluormethyl-pyrimidin, 2-(2-Methoxy-4-piperazin-1-yl-phenylamino)-4-((S)-4-oxo-2,3,10,10a-tetrahydro-1*H*,4*H-*9-oxa-3a-aza-benzo[*f*]azulen-5-ylamino-5-trifluormethyl-pyrimidin, 2-[4-(4-Ethyl-piperazin-1-yl)-2-methoxy-phenylamino]-4-((S)-4-oxo-2,3,10,10a-tetrahydro-1*H*,4*H*-9-oxa-3a-aza-benzo[*f*]azulen-5-ylamino-5-trifluormethyl-pyrimidin, 2-[4-(4-Methyl-piperazin-1-yl)-2-methoxy-phenylamino]-4-((S)-4-oxo-2,3,10,10a-tetrahydro-1*H*,4*H*-9-oxa-3a-aza-benzo[*f*]azulen-5-ylamino-5-trifluormethyl-pyrimidin, 3,3-Dimethyl-6-{2-[4-(4-methyl-piperazin-1-yl)-phenylamino]-5-trifluormethyl-pyrimidin-4-ylamino}-3,4-dihydro-2*H*-benzo[*f*][1,4]oxazepin-5-on, 6-{2-[4-(4-Ethyl-piperazin-1-yl)-phenylamino]-5-trifluormethyl-pyrimidin-4-ylamino}-3,3-dimethyl-3,4-dihydro-2*H*-benzo[*f*][1,4]oxazepin-5-on 3,3-Dimethyl-6-{2-[4-(4-prop-2-ynyl-piperazin-1-yl)-phenylamino]-5-trifluormethyl-pyrimidin-4-ylamino}-3,4-dihydro-2*H*-benzo[*f*][1,4]oxazepin-5-on, 6- {2-[4-(4-Isopropyl-piperazin-1-yl)-phenylamino]-5-trifluormethyl-pyrimidin-4-ylamino}-3,3-dimethyl-3,4-dihydro-2*H*-benzo[*f*][1,4]oxazepin-5-on, (S)-3-Methyl-6-[2-(4-piperazin-1-yl-phenylamino)-5-trifluormethyl-pyrimidin-4-ylamino]-3,4-dihydro-2*H*-benzo[*f*][1,4]oxazepin-5-on, (S)-3-Methyl-6-{2-[4-(4-methyl-piperazin-1-yl)-phenylamino]-5-trifluormethyl-pyrimidin-4-ylamino}-3,4-dihydro-2*H*-benzo[*f*][1,4]oxazepin-5-on, (S)-6-{2-[4-(4-Isopropyl-piperazin-1-yl)-phenylamino]-5-trifluormethyl-pyrimidin-4-ylamino}-3-methyl-3,4-dihydro-2*H*-benzo[*f*][1,4]oxazepin-5-on, (S)-3-Methyl-6-{2-[4-(4-prop-2-ynyl-piperazin-1-yl)-phenylamino]-5-trifluormethyl-pyrimidin-4-ylamino}-3,4-dihydro-2*H*-benzo[*f*][1,4]oxazepin-5-on (S)-3-Ethyl-6-[2-(4-piperazin-1-yl-phenylamino)-5-trifluormethyl-pyrimidin-4-ylamino]-3,4-dihydro-2*H*-benzo[*f*][1,4]oxazepin-5-on, (S)-6-[2-(4-Piperazin-1-yl-phenylamino)-5-trifluormethyl-pyrimidin-4-ylamino]-3-propyl-3,4-dihydro-2*H*-benzo[*f*][1,4]oxazepin-5-on, (S)-6-{2-[4-(4-Prop-2-ynyl-piperazin-1-yl)-phenylamino]-5-trifluormethyl-pyrimidin-4-ylamino}-1,2,3,11a-tetrahydro-10*H*-benzo[*e*]pyrrolo[1,2-*a*][1,4]diazepin-5,11-dion, (S)-6-{2-[4-(4-Ethyl-piperazin-1-yl)-phenylamino]-5-trifluormethyl-pyrimidin-4-ylamino}-1,2,3,11a-tetrahydro-10*H*-benzo[*e*]pyrrolo[1,2-*a*][1,4]diazepin-5,11-dion, (S)-6-{2-[4-(4-Isobutyl-piperazin-1-yl)-phenylamino]-5-trifluormethyl-pyrimidin-4-ylamino}-1,2,3,11a-tetrahydro-10*H*-benzo[*e*]pyrrolo[1,2-*a*][1,4]diazepin-5,11-dion, (S)-6-{2-[4-(4-Methyl-piperazin-1-yl)-phenylamino]-5-trifluormethyl-pyrimidin-4-ylamino}-1,2,3,11a-tetrahydro-10*H*-benzo[*e*]pyrrolo[1,2-*a*][1,4]diazepin-5,11-dione, 6-{2-[4-(4-Ethyl-piperazin-1-yl)-phenylamino]-5-trifluormethyl-pyrimidin-4-ylamino}-3,3-dimethyl-3,4-dihydro-1*H*-benzo[*e*][1,4]diazepin-2,5-dion, 3,3-Dimethyl-6-{2-[4-(4-methyl-piperazin-1-yl)-phenylamino]-5-trifluormethyl-pyrimidin-4-ylamino}-3,4-dihydro-1*H*-benzo[*e*][1,4]diazepin-2,5-dion, 6-{2-[4-(4-Isopropyl-piperazin-1-yl)-phenylamino]-5-trifluormethyl-pyrimidin-4-ylamino}-3,3-dimethyl-3,4-dihydro-1*H*-benzo[*e*][1,4]diazepin-2,5-dion, 3,3-Dimethyl-6-{2-[4-(4-prop-2-ynyl-piperazin-1-yl)-phenylamino]-5-trifluormethyl-pyrimidin-4-ylamino}-3,4-dihydro-1*H*-benzo[*e*][1,4]diazepin-2,5-dion, (S)-6-{2-[4-(4-Isopropyl-piperazin-1-yl)-phenylamino]-5-trifluormethyl-pyrimidin-4-ylamino}-1,2,3,11a-tetrahydro-10*H*-benzo[*e*]pyrrolo[1,2-*a*][1,4]diazepin-5,11-dion, (S)-6-{2-[4-(4-Isopropyl-piperazin-1-yl)-phenylamino]-5-trifluormethyl-pyrimidin-4-ylamino}-3-methyl-3,4-dihydro-1*H*-benzo[*e*][1,4]diazepin-2,5-dion, (S)-3-Methyl-6-{2-[4-(4-methyl-piperazin-1-yl)-phenylamino]-5-trifluormethyl-pyrimidin-4-ylamino}-3,4-dihydro-1*H*-benzo[*e*][1,4]diazepin-2,5-dion, (S)-3-Methyl-6-{2-[4-(4-prop-2-ynyl-piperazin-1-yl)-phenylamino]-5-trifluormethyl-pyrimidin-4-ylamino}-3,4-dihydro-1*H*-benzo[*e*][1,4]diazepin-2,5-dion, (S)-6-{2-[4-(4-Ethyl-piperazin-1-yl)-phenylamino]-5-trifluormethyl-pyrimidin-4-ylamino}-3-methyl-3,4-dihydro-1*H*-benzo[*e*][1,4]diazepin-2,5-dion und {(S)-2,5-Dioxo-6-[2-(4-piperazin-1-yl-phenylamino)-5-trifluormethyl-pyrimidin-4-ylamino]-2,3,4,5-tetrahydro-1*H*-benzo[*e*][1,4]diazepin-3-yl}-essigsäuremethylester ausgenommen sind.

Ein Aspekt der Erfindung sind Verbindungen der allgemeinen Formel (1), worin R⁵ Halogen oder -CF₃ bedeutet.

Ein weiterer Aspekt der Erfindung sind Verbindungen der allgemeinen Formel (1), worin
- X: Sauerstoff und
- Y: -CH₂- bedeuten.

Ein weiterer Aspekt der Erfindung sind Verbindungen der allgemeinen Formel (1), worin
- X: Stickstoff und
- Y: C=O bedeuten.

(A) Aspekte bezüglich R^{1'}
   (A1) Ein Aspekt der Erfindung sind Verbindungen der allgemeinen Formel (1), worin R^{1'} C₁₋₅Alkyl und m = 1 bedeutet.
   (A2) Ein Aspekt der Erfindung sind Verbindungen der allgemeinen Formel (1), worin R^{1'} C₁₋₅Alkyl und m = 2 bedeutet.
   (A3) Ein Aspekt der Erfindung sind Verbindungen der allgemeinen Formel (1), worin die beiden R^{1'} gemeinsam einen Cycloalkylrest bilden.
   (A4) Ein weiterer Aspekt der Erfindung sind Verbindungen der allgemeinen Formel (1), worin R¹ mit einem R^{1'} einen Cyclopentylring bilden.
(B) Aspekte bezüglich R² und R³
   (B1) Ein Aspekt der Erfindung sind Verbindungen der allgemeinen Formel (1), worin R² eine gegebenenfalls einfach substituierte Piperidinylgruppe bedeutet.
   (B2) Ein weiterer Aspekt der Erfindung sind Verbindungen der allgemeinen Formel (1), worin R² eine am Stickstoff substituierte Piperidinylgruppe bedeutet.
   (B3) Ein weiterer Aspekt der Erfindung sind Verbindungen der allgemeinen Formel (1), worin R² eine am Stickstoff mit Methyl, Ethyl oder Cyclopropylmethyl substituierte Piperidinylgruppe bedeutet.
   (B4) Ein weiterer Aspekt der Erfindung sind Verbindungen der allgemeinen Formel (1), worin R² und R³ gemeinsam eine gegebenenfalls einfach oder mehrfach substituierte Piperazinylgruppe bilden.
   (B5) Ein weiterer Aspekt der Erfindung sind Verbindungen der allgemeinen Formel (1), worin R² und R³ gemeinsam eine am Stickstoff substituierte Piperazinylgruppe bedeutet.
   (B6) Ein weiterer Aspekt der Erfindung sind Verbindungen der allgemeinen Formel (1), worin R² und R³ gemeinsam eine am Stickstoff mit Methyl, Ethyl, Isopropyl oder Cyclopropylmethyl substituierte Piperazinylgruppe bedeuten.
(C) Aspekte bezüglich X und Y
   (C1) Ein Aspekt der Erfindung sind Verbindungen der allgemeinen Formel (1), worin X Sauerstoff und Y -CH₂- bedeutet.
   (C2) Ein weiterer Aspekt der Erfindung sind Verbindungen der allgemeinen Formel (1), worin X Stickstoff und Y C=O bedeutet.

Alle der oben genannten Aspekte (A1) bis (A4) für R^{1'}, (B1) bis (B6) für R² und R³ und (C1) und (C2) für X und Y können beliebig miteinander kombiniert werden.

Die nachfolgende Tabelle stellt bevorzugte Kombinationen verschiedener Aspekte der erfindungsgemäßen Verbindungen der Formel (1) dar:

| Ausführungsform | R^{1'} | R² und R³ | X und Y |
|---|---|---|---|
| I-1 | A1 | B1 | C1 |
| I-2 | A1 | B2 | C1 |
| I-3 | A1 | B3 | C1 |
| I-4 | A1 | B4 | C1 |
| I-5 | A1 | B5 | C1 |
| I-6 | A1 | B6 | C1 |
| I-7 | A2 | B4 | C1 |
| I-8 | A2 | B5 | C1 |
| I-9 | A2 | B6 | C1 |
| I-10 | A3 | B4 | C2 |
| I-11 | A4 | B4 | C1 |
| I-12 | A4 | B5 | C1 |
| I-13 | A4 | B6 | C1 |
| I-14 | A4 | B4 | C2 |
| I-15 | A4 | B5 | C2 |
| I-16 | A4 | B6 | C2 |

Ein weiterer Aspekt der Erfindung sind Verbindungen der allgemeinen Formel (1) - oder deren pharmakologisch verträglichen Salze - als Arzneimittel.

Ein weiterer Aspekt der Erfindung sind Verbindungen der allgemeinen Formel (1) - oder deren pharmakologisch verträglichen Salze - zur Herstellung eines Arzneimittels mit antiproliferativer Wirkung.

Ein weiterer Aspekt der Erfindung sind Verbindungen der allgemeinen Formel (1) - oder deren pharmakologisch verträglichen Salze - zur Herstellung eines Arzneimittels mit antiproliferativer Wirkung mit einem selektiven kinaseinhibierendem Wirkmechanismus.

Ein weiterer Aspekt der Erfindung ist die Verwendung einer Verbindung der allgemeinen Formel 1 zur Herstellung eines Arzneimittels zur Behandlung und/oder Prävention von Krebs, Infektionen, Entzündungs- und Autoimmunerkrankungen.

Ein weiterer Aspekt der Erfindung ist eine pharmazeutische Zubereitung, enthaltend als Wirkstoff eine oder mehrere Verbindungen der allgemeinen Formel (1), oder deren pharmakologisch verträglichen Salze, gegebenenfalls in Kombination mit üblichen Hilfs- und/oder Trägerstoffen.

Ein weiterer Aspekt der Erfindung ist eine pharmazeutische Präparation umfassend eine Verbindung der allgemeinen Formel (1), gegebenenfalls in Form ihrer Tautomeren, ihrer Racemate, ihrer Enantiomere, ihrer Diastereomere und ihrer Gemische, sowie gegebenenfalls ihrer pharmakologisch verträglichen Salze, und mindestens eine weitere zytostatische oder zytotoxische Wirksubstanz.

### Definitionen

Wie hierin verwendet treffen folgenden Definitionen zu, falls nicht anders beschrieben.

Unter Alkyl-Substitutenten sind jeweils gesättigte, ungesättigte, unverzweigte oder verzweigte aliphatische Kohlenwasserstoffreste (Alkylrest) zu verstehen und umfasst sowohl gesättigte Alkylreste als auch ungesättigte Alkenyl- und Alkinylreste. Alkenyl-Substituenten sind jeweils unverzweigt oder verzweigte, ungesättigte Alkylreste, die mindestens eine Doppelbindung aufweisen. Unter Alkinyl-Substituenten sind jeweils unverzweigt oder verzweigte, ungesättigte Alkylreste, die mindestens eine Dreifachbindung aufweisen, zu verstehen.

Heteroalkyl repräsentiert unverzweigt oder verzweigte aliphatische Kohlenwasserstoffketten, die 1 bis 3 Heteroatome enthalten, wobei jedes der verfügbaren Kohlenstoff- und Heteroatome in der Heteroalkylkette gegebenenfalls jeweils unabhängig voneinander substituiert sein kann und die Heteroatome unabhängig voneinander ausgewählt werden aus der Gruppe bestehend aus O, N, P, PO, PO₂, S, SO und SO₂ (z. B. Dimethylaminomethyl, Dimethylaminoethyl, Dimethylaminopropyl, Diethylaminomethyl, Diethylaminoethyl, Diethylaminopropyl, 2-Disopropylaminoethyl, Bis-2-methoxyethylamino, [2-(Dimethylamino-ethyl)-ethyl-amino]-methyl, 3-[2-(Dimethylamino-ethyl)-ethyl-amino]-propyl, Hydroxymethyl, 2-Hydroxyethyl, 3-Hydroxypropyl, Methoxy, Ethoxy, Propoxy, Methoxymethyl, 2-Methoxyethyl).

Halogen bezieht sich auf Fluor-, Chlor-, Brom- und/oder Jodatome.

Halogenalkyl bezieht sich auf Alkylreste, in denen ein oder mehrere Wasserstoffatome durch Halogenatome ersetzt sind. Halogenalkyl umfasst sowohl gesättigte Alkylreste als auch ungesättigte Alkenyl- und Alkinylreste, wie beispielsweise -CF₃, -CHF₂, -CH₂F, -CF₂CF₃,-CHFCF₃, -CH₂CF₃, -CF₂CH₃, -CHFCH₃, -CF₂CF₂CF₃, -CF₂CH₂CH₃, -CF=CF₂, -CCl=CH₂, -CBr=CH₂, -CJ=CH₂, -C≡C-CF₃, -CHFCH₂CH₃ und -CHFCH₂CF3.

Unter Pseudohalogen sind folgende Reste zu verstehen: -OCN, -SCN, -CF3 und -CN.

Unter Cycloalkyl ist ein mono- oder polyzyklischer Ring zu verstehen, wobei das Ringsystem ein gesättigter Ring aber auch ein ungesättigter, nicht aromatischer Ring beziehungsweise eine Spiroverbindung sein kann, welcher gegebenenfalls auch Doppelbindungen enthalten kann, wie zum Beispiel Cyclopropyl, Cyclopropenyl, Cyclobutyl, Cyclobutenyl, Cyclopentyl, Cyclopentenyl, Cyclohexyl, Cyclohexenyl, Cycloheptanyl, Cycloheptenyl, Norbomyl, Norbornenyl, Indanyl, Adamantyl Spiroheptanyl und Spiro[4.2]heptanyl.

Cycloalkylalkyl umfasst eine nicht-zyklische Alkylgruppe, in der ein an einem Kohlenstoffatom gebundenes Wasserstoffatom durch eine Cycloalkylgruppe ersetzt ist.

Aryl bezieht sich auf monozyklische oder bizyklische Ringe mit 6-12 Kohlenstoffatomen wie beispielsweise Phenyl und Naphthyl.

Arylalkyl umfasst eine nicht-zyklische Alkylgruppe, in der ein an einem Kohlenstoffatom gebundenes Wasserstoffatom durch eine Arylgruppe ersetzt ist.

Unter Heteroaryl sind mono- oder polyzyklische Ringe zu verstehen, welche anstelle eines oder mehrere Kohlenstoffatome ein oder mehrere, gleich oder verschiedene Heteroatome enthalten, wie z.B. Stickstoff-, Schwefel- oder Sauerstoffatome. Beispielsweise genannt seien Furyl, Thienyl, Pyrrolyl, Oxazolyl, Thiazolyl, Isoxazolyl, Isothiazolyl, Pyrazolyl, Imidazolyl, Triazolyl, Tetrazolyl, Oxadiazolyl, Thiadiazolyl, Pyridyl, Pyrimidyl, Pyridazinyl, Pyrazinyl und Triazinyl. Beispiele für bizyklische Heteroarylreste sind Indolyl, Isoindolyl, Benzofuranyl, Benzothienyl, Benzoxazolyl, Benzothiazolyl, Benzisoxazolyl, Benzisothiazolyl, Benzimidazolyl, Indazolyl, Isoquinolinyl, Quinolinyl, Quinoxalinyl, Cinnolinyl, Phthalazinyl, Quinazolinyl und Benzotriazinyl, Indolizinyl, Oxazolopyridinyl, Imidazopyridinyl, Naphthyridinyl, Indolinyl, Isochromanyl, Chromanyl, Tetrahydroisochinolinyl, Isoindolinyl, Isobenzotetrahydrofuranyl, Isobenzotetrahydrothienyl, Isobenzothienyl, Benzoxazolyl, Pyridopyridinyl, Benzotetrahydrofuranyl, Benzotetrahydrothienyl, Purinyl, Benzodioxolyl, Triazinyl, Phenoxazinyl, Phenothiazinyl, Pteridinyl, Benzothiazolyl, Imidazopyridinyl, Imidazothiazolyl, Dihydrobenzisoxazinyl, Benzisoxazinyl, Benzoxazinyl, Dihydrobenzisothiazinyl, Benzopyranyl, Benzothiopyranyl, Coumarinyl, Isocoumarinyl, Chromonyl, Chromanonyl, Pyridinyl-*N*-oxid Tetrahydroquinolinyl, Dihydroquinolinyl, Dihydroquinolinonyl, Dihydroisoquinolinonyl, Dihydrocoumarinyl, Dihydroisocoumarinyl, Isoindolinonyl, Benzodioxanyl, Benzoxazolinonyl, Pyrrolyl-*N-*oxid, Pyrimidinyl-*N*-oxid, Pyridazinyl-*N*-oxid, Pyrazinyl-*N*-oxid, Quinolinyl-*N*-oxid, Indolyl-*N*-oxid, Indolinyl-*N*-oxid, Isoquinolyl-*N*-oxid, Quinazolinyl-*N*-oxid, Quinoxalinyl-*N*-oxid, Phthalazinyl-*N*-oxid, Imidazolyl-*N*-oxid, Isoxazolyl-*N*-oxid, Oxazolyl-*N*-oxid, Thiazolyl-*N*-oxid, Indolizinyl-*N*-oxid, Indazolyl-*N*-oxid, Benzothiazolyl-*N*-oxid, Benzimidazolyl-*N*-oxid, Pyrrolyl-*N*-oxid, Oxadiazolyl-*N*-oxid, Thiadiazolyl-*N*-oxid, Triazolyl-*N*-oxid, Tetrazolyl-*N*-oxid, Benzothiopyranyl-*S*-oxid und Benzothiopyranyl-*S,S-*dioxid.

Heteroarylalkyl umfasst eine nichtzyklische Alkylgruppe, in der ein an einem Kohlenstoffatom gebundenes Wasserstoffatom durch eine Heteroarylgruppe ersetzt ist.

Heterocycloalkyl bezieht sich auf 3 - 12 Kohlenstoffatome umfassende gesättigte oder ungesättigte, nicht aromatische mono-, polyzyklische oder überbrückte polyzyklische Ringe oder Spiroverbindungen, welche anstelle eines oder mehrere Kohlenstoffatome Heteroatome, wie Stickstoff, Sauerstoff oder Schwefel, tragen. Beispiele für solche Heterocylylreste sind Tetrahydrofuranyl, Pyrrolidinyl, Pyrrolinyl, Imidazolidinyl, Imidazolinyl, Pyrazolidinyl, Pyrazolinyl, Piperidinyl, Piperazinyl, Indolinyl, Isoindolinyl, Morpholinyl, Thiomorpholinyl, Homomorpholinyl, Homopiperidinyl, Homopiperazinyl, Homothiomorpholinyl, Thiomorpholinyl-S-oxid, Thiomorpholinyl-S,S-dioxid, Tetrahydropyranyl, Tetrahydrothienyl, Homothiomorpholinyl-S,S-Dioxid, Oxazolidinonyl, Dihydropyrazolyl, Dihydropyrrolyl, Dihydropyrazinyl, Dihydropyridinyl, Dihydropyrimidinyl, Dihydrofuryl, Dihydropyranyl, Tetrahydrothienyl-S-oxid, Tetrahydrothienyl-S,S-dioxid, Homothiomorpholinyl-S-oxid, 2-Oxa-5-azabicyclo[2.2.1]heptan, 8-Oxa-3-aza-bicyclo[3.2.1]octan, 3,8-Diaza-bicyclo[3.2.1]octan, 2,5-Diaza-bicyclo[2.2.1]heptan, 3,8-Diaza-bicyclo[3.2.1]octan, 3,9-Diaza-bicyclo[4.2.1]nonan und 2,6-Diaza-bicyclo[3.2.2]nonan.

Heterocycloalkylalkyl bezieht sich auf eine nicht-zyklische Alkylgruppe, in der ein an einem Kohlenstoffatom gebundenes Wasserstoffatom durch eine Heterocycloalkylgruppe ersetzt ist.

### Herstellung der erfindungsgemäßen Verbindungen

Die Herstellung der erfindungsgemäßen Verbindungen kann nach den, im nachfolgenden Text beschriebenen Syntheseverfahren A oder B erfolgen, wobei die Substituenten der allgemeinen Formeln (I bis VI) die zuvor genannten Bedeutungen haben. Die als Ausgangsstoffe verwendeten Verbindungen sind teilweise literaturbekannt oder man erhält diese nach literaturbekannten Verfahren. Diese Verfahren sind als Erläuterung der Erfindung zu verstehen, ohne selbige auf deren Gegenstand zu beschränken.

### Verfahren A

### Stufe 1A

Die Herstellung der Zwischenverbindung III erfolgt durch Substitution einer Abgangsgruppe LG, beispielsweise Halogen, SCN oder Methoxy, vorzugsweise Chlor, an einem heteroaromatischem System I durch ein Nukleophil II.

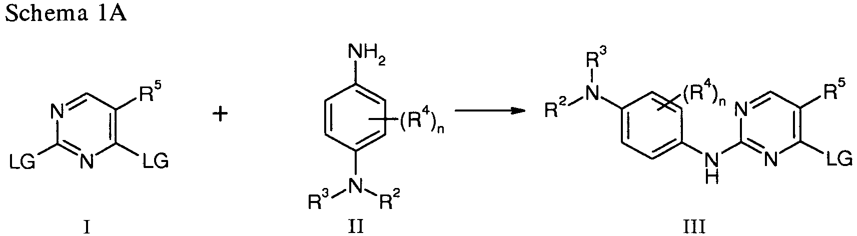

Es werden 1 Äquivalent der Verbindung I und 1 bis 1.5 Äquivalente der Verbindung II in einem Lösungsmittel, beispielsweise 1,4-Dioxan, Tetrahydrofuran, *N,N-*Dimethylformamid oder *N,N*-Dimethylacetamid gerührt. Bei einer Temperatur von 15 bis 25 °C werden 2 bis 2.5 Äquivalente einer Base, beispielsweise Kaliumcarbonat, Natriumcarbonat, Cäsiumcarbonat, *N*-Ethyl-*N,N*-diisopropylamin oder Triethylamin zugegeben. Das Reaktionsgemisch wird 12 bis 72 h bei einer Temperatur von 15 bis 25°C weitergerührt. Danach wird das Lösungsmittel abdestilliert und der Rückstand mit Wasser versetzt, welches mit einer Mineralsäure, beispielsweise Salzsäure oder Schwefelsäure auf einen sauren pH-Wert kleiner 4 eingestellt wird. Dieses Gemisch wird zwei- bis dreimal mit einem organischen Lösungsmittel beispielsweise Diethylether, Ethylacetat oder Dichlormethan extrahiert. Die vereinigten organischen Extrakte werden getrocknet und das Lösungsmittel abdestilliert. Der Rückstand wird chromatographisch gereinigt.

### Stufe 2A

Die Herstellung der Endverbindung V erfolgt durch Substitution einer Abgangsgruppe LG, beispielsweise Halogen, SCN oder Methoxy, vorzugsweise Chlor, an einem heteroaromatischen System III durch ein Nukleophil IV.

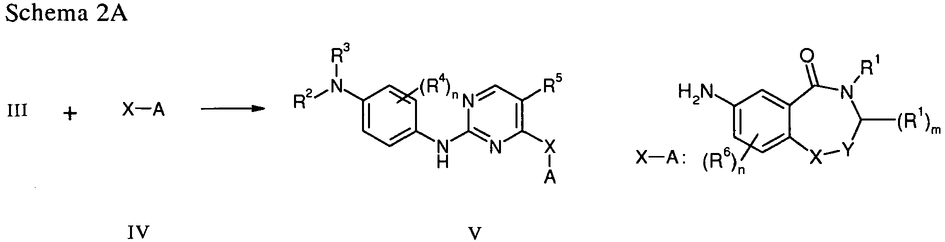

Es werden 1 Äquivalent der Verbindung III und 1 bis 3 Äquivalente der Verbindung IV in einem Lösungsmittel, beispielsweise 1,4-Dioxan, *N,N*-Dimethylformamid, *N,N-*Dimethylacetamid oder *N*-Methyl-2-pyrrolidinon gerührt. Bei einer Temperatur von 15 bis 40 °C werden 1 bis 2 Äquivalente einer Mineralsäure, beispielsweise Schwefelsäure oder Salzsäure, zugegeben. Das Reaktionsgemisch wird 12 bis 72 h bei einer Temperatur von 20 bis 100 °C weitergerührt. Danach wird das Lösungsmittel abdestilliert und der Rückstand chromatographisch gereinigt.

### Verfahren B

### Stufe 1B

Die Herstellung der Zwischenverbindung VI erfolgt durch Substitution einer Abgangsgruppe LG, beispielsweise Halogen, SCN oder Methoxy, vorzugsweise Chlor, an einem heteroaromatischem System I durch ein Nukleophil IV.

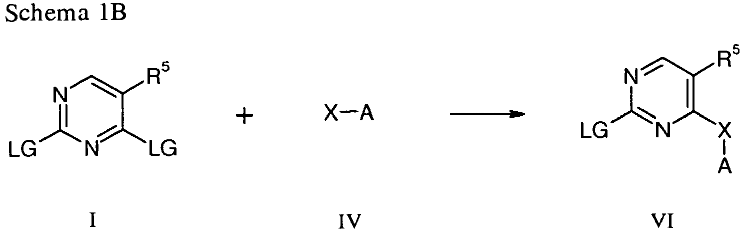

Es werden 1 Äquivalent der Verbindung I und 1 bis 3 Äquivalente einer Base, beispielsweise Triethylamin oder Ethyldiisopropylamin, in einem Lösungsmittel, beispielsweise 1,4-Dioxan, Tetrahydrofuran, *N,N*-Dimethylformamid oder *N,N-*Dimethylacetamid gerührt. Bei einer Temperatur von -60 bis 0 °C werden 0.8 bis 1.5 Äquivalente einer Verbindung IV zugegeben. Das Reaktionsgemisch wird 12 bis 72 h bei einer Temperatur von 15 bis 25 °C weitergerührt. Danach wird das Lösungsmittel abdestilliert und der Rückstand chromatographisch gereinigt.

### Stufe 2B

Die Herstellung der Endverbindung V erfolgt durch Substitution einer Abgangsgruppe LG, beispielsweise Halogen, SCN oder Methoxy, vorzugsweise Chlor, an einem heteroaromatischen System VI durch ein Nukleophil II.

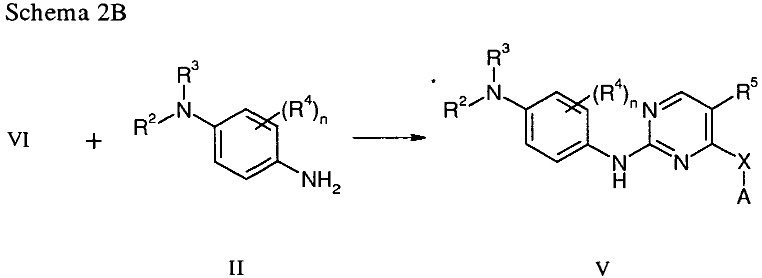

Es werden 1 Äquivalent der Verbindung VI und 1 bis 1:5 Äquivalente der Verbindung II in einem Lösungsmittel, beispielsweise 1,4-Dioxan, *N,N*-Dimethylformamid, *N,N-*Dimethylacetamid oder *N*-Methyl-2-pyrrolidinon, gerührt. Bei einer Temperatur von 15 bis 40 °C werden 1 bis 2 Äquivalente einer Säure, beispielsweise Schwefelsäure oder Salzsäure, zugegeben. Das Reaktionsgemisch wird 12 bis 72 h bei einer Temperatur von 20 bis 100 °C weitergerührt. Danach wird das Lösungsmittel abdestilliert und der Rückstand chromatographisch gereinigt.

### Methode 1

### (S)-6-Amino-3-ethyl-3,4-dihydro-2H-benzo[f][1,4]oxazepin-5-on

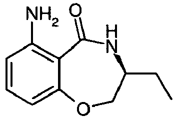

### a) 2-Amino-6-(2-(S)-aminobutoxy)-benzonitril

5.01 g (51.3 mmol) (S)-2-Amino-1-butanol werden in 30 mL 1,4-Dioxan gelöst, mit 2.10 g (52.5 mmol) Natriumhydrid versetzt und 30 min bei Raumtemperatur gerührt. Diesem Reaktionsgemisch werden 5.00 g (36.7 mmol) 2-Amino-6-fluorbenzonitril beigefügt und 24 h bei 50 °C gerührt. Anschließend wird das Lösungsmittel im Vakuum entfernt und das Rohprodukt durch Chromatographie gereinigt. Als Trägermaterial dient Kieselgel und als Eluent wird Dichlormethan, dem 5 % einer Mischung aus 90 % Methanol und 10 % gesättigter wässriger Ammoniak-Lösung zugefügt sind, verwendet.

| | |
|---|---|
| Ausbeute: | 5.55 g |
| MS (ESI): | 206 (M+H)⁺ |

### b) 2-Amino-6-(2-(S)-aminobutoxy)-benzoesäure

5.53 g (27.05 mmol) (S)-2-Amino-6-(1-aminomethyl-propoxy)-benzonitril werden in 70 mL 20 %ige ethanolische KOH gelöst und 5 d bei 100 °C gerührt. Anschließend wird das Lösungsmittel im Vakuum entfernt und das Rohprodukt durch Chromatographie gereinigt. Als Trägermaterial dient Kieselgel und als Eluent wird Dichlormethan, dem 12 % einer Mischung aus 90 % Methanol und 10 % gesättigter wässriger Ammoniak-Lösung zugefügt sind, verwendet.

| | |
|---|---|
| Ausbeute: | 4.76 g |
| MS (ESI): | 225 (M+H)⁺ |

### c) (S)-6-Amino-3-ethyl-3,4-dihydro-2H-benzo[f][1,4]oxazepin-5-on

4.76 g (21.23 mmol) (S)-2-Amino-6-(1-aminomethyl-propoxy)-benzoesäure werden in 600 mL Tetrahydrofuran gelöst, mit 12.5 g (63.7 mmol) 1-(3-Dimethyl-aminopropyl)-3-ethylcarbodiimid hydrochlorid und 11.11 ml (63.7 mmol) Diisopropyl-ethylamin versetzt und 3 h bei 50 °C gerührt. Anschließend wird das Lösungsmittel im Vakuum entfernt und das Rohprodukt durch Chromatographie gereinigt. Als Trägermaterial dient Kieselgel und als Eluent wird Dichlormethan, dem 4 % einer Mischung aus 90 % Methanol und 10 % gesättigter wässriger Ammoniak-Lösung zugefügt sind, verwendet.

| | |
|---|---|
| Ausbeute: | 1.839g |
| MS (ESI): | 207 (M+H)⁺ |

Analog zu dieser Methode werden folgende Verbindungen hergestellt. Dabei ersetzt man (S)-1-Amino-2-butanol durch einen entsprechenden Aminoalkohol.

| | MS (ESI) (M+H)⁺ |
|---|---|
| 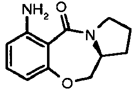 | 219 |
| 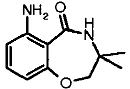 | 207 |

### Methode 2

### (R)-6-Amino-11a-methyl-1,2,3,11a-tetrahydro-10H-benzo[e]pyrrolo[1,2-a][1,4]diazepin-5,11-dion

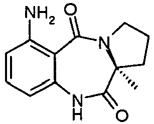

### a) (R)-1-(2-Amino-6-nitro-benzoyl)-2-methyl-pyrrolidin-2-carbonsäuremethylester

1.18 g (6,5 mmol) 2-Amino-6-nitrobenzoesäure, 0.83 g (4,6 mmol) (R)-2-Methylpyrrolidin-2-methylester hydrochlorid, 4.05 mL (23,2 mmol) N-Ethyldiisopropylamin werden mit 2.5 ml Tetrahydrofuran versetzt. Diesem Reaktionsgemisch werden 1.71 g (5.1 mmol) *O*-(Benzotriazol-1-yl)-*N,N,N',N'*-tetramethyluronium-tetrafluorborat zugesetzt und 12 h bei 50 °C erhitzt. Anschließend wird das Lösungsmittel im Vakuum entfernt und das Rohprodukt durch Chromatographie gereinigt. Als Trägermaterial dient Kieselgel und als Eluent wird ein Gemisch bestehend aus Cyclohexan : Ethylacetat (50 : 50) verwendet.

| | |
|---|---|
| Ausbeute: | 930 mg |
| MS (ESI): | 308 (M+H)⁺ |

### b) (R)-1-(2-Amino-6-nitro-benzoyl)-2-methyl-pyrrolidin-2-carbonsäure

930 mg (3.02 mmol) (R)-1-(2-Amino-6-nitro-benzoyl)-2-methyl-pyrrolidin-2-carbonsäuremethylester werden in 3 mL 20 % ige ethanolische KOH gelöst und 1.5 h bei 50 °C gerührt. Anschließend wird das Lösungsmittel im Vakuum entfernt und das Rohprodukt durch Chromatographie gereinigt. Als Trägermaterial dient Kieselgel und als Eluent wird Dichlormethan, dem 15 % einer Mischung aus 90 % Methanol und 10 % gesättigter wässriger Ammoniak-Lösung zugefügt sind, verwendet.

| | |
|---|---|
| Ausbeute: | 557 mg |
| MS(ESI): | 294 (M+H)⁺ |

### c) (R)-1-(2,6-Diamino-benzoyl)-2-methyl-pyrrolidin-2-carbonsäure

366 mg (1.25 mmol) (R)-1-(2-Amino-6-nitro-benzoyl)-2-methyl-pyrrolidin-2-carbonsäure werden in 50 mL Methanol gelöst und mit 40 mg Palladium auf Kohle (10 % Pd) versetzt. Das Reaktionsgemisch wird 9 h bei 5 bar Wasserstoff-Druck und 25 °C hydriert. Danach wird der Katalysator abfiltriert, das Lösungsmittel im Vakuum entfernt und das Rohprodukt durch Chromatographie gereinigt. Als Trägermaterial dient C18-RP-Kieselgel und es wird ein Gradient durchlaufen der am Startpunkt aus 95 % Wasser und 5 % Acetonitril und am Endpunkt aus 5 % Wasser und 95 % Acetonitril besteht.

| | |
|---|---|
| Ausbeute: | 76 mg |
| MS (ESI): | 264 (M+H)⁺ |

### d) (R)-6-Amino-11a-methyl-1,2,3,11a-tetrahydro-10H-benzo[e]pyrrolo[1,2-a][1,4] diazepin-5,11-dion

76 mg (0,29 mmol) 2(R)-1-(2,6-Diamino-benzoyl)-2-methyl-pyrrolidin-2-carbonsäure werden in 2 mL Tetrahydrofuran gelöst, mit 143 mg (0.9 mmol) 1-(3-Dimethylaminopropyl)-3-ethylcarbodiimid hydrochlorid und 103 µL (0.6 mmol) Diisopropyl-ethylamin versetzt und 17 h bei 50 °C gerührt. Anschließend wird das Lösungsmittel im Vakuum entfernt und das Rohprodukt durch Chromatographie gereinigt. Als Trägermaterial dient Kieselgel und als Eluent wird Dichlormethan, dem 5 % einer Mischung aus 90 % Methanol und 10 % gesättigter wässriger Ammoniaklösung zugefügt sind, verwendet.

| | |
|---|---|
| Ausbeute: | 20 mg |
| MS (ESI): | 246 (M+H)⁺ |

Analog dazu werden folgende Verbindungen hergestellt:

| | MS (ESI) (M+H)⁺: |
|---|---|
| 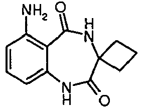 | 232 |
| 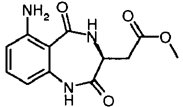 | 264 |

### Methode 3

### 4-(4-Amino-phenyl)-piperazin-1-carbonsäurebenzylester

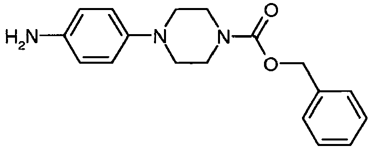

### a) 4-(4-Nitro-phenyl)-piperazin-1-carbonsäurebenzylester

3.76 ml (35.082 mmol) 4-Fluornitrobenzol werden in 40 mL DMA gelöst und mit 9.25 mL (52.62 mmol) N-Ethyldiisopropylamin und 7.6 ml (38.59 mmol) 1-(Benzyloxycarbonyl)-piperazin versetzt. Die Reaktionsmischung wird 18 h bei 80 °C gerührt. Anschließend wird das Lösungsmittel im Vakuum entfernt und das Rohprodukt durch Chromatographie gereinigt. Als Trägermaterial dient Kieselgel und als Eluent wird ein Gemisch bestehend aus Cyclohexan : Ethylacetat (50 : 50) verwendet.

| | |
|---|---|
| Ausbeute: | 11.28 g |
| MS (ESI): | 342 (M+H)⁺ |

### b) 4-(4-Amino-phenyl)-piperazin-1-carbonsäurebenzylester

11.28 g (33.04 mmol) 4-(4-Nitro-phenyl)-piperazin-1-carbonsäurebenzylester werden in 500 mL Methanol gelöst und mit 1 g Raney Nickel versetzt. Es wird 18 h bei 5 bar Wasserstoffdruck hydriert. Anschließend filtriert man vom Katalysator ab versetzt mit 70 mL einer wässrigen 1 N Salzsäure und entfernt das Lösungsmittel im Vakuum.

| | |
|---|---|
| Ausbeute: | 10.89 g |
| MS (ESI): | 312(M+H)⁺ |

Analog zu diesem Verfahren werden folgende Verbindungen hergestellt:

| | MS (ESI) (M+H)⁺ | | MS (ESI) (M+H)⁺ |
|---|---|---|---|
| 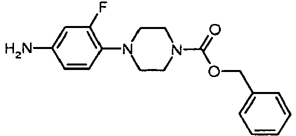 | 330 | 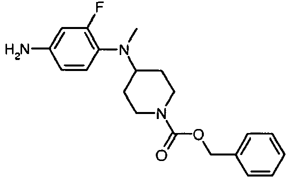 | 358 |
| 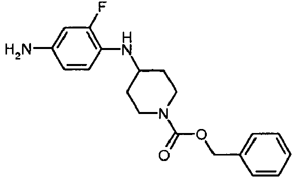 | 344 | 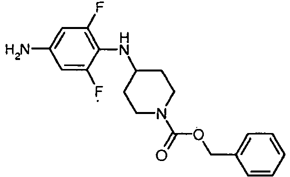 | 362 |
| 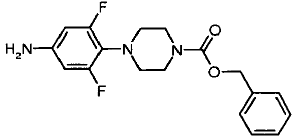 | 348 | 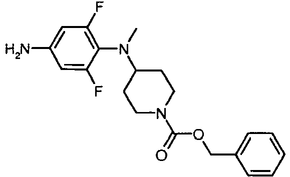 | 376 |

### Chromatographie

Für die Mitteldruck Chromatographie (MPLC) wird Kieselgel der Firma Millipore (Bezeichnung: Granula Silica Si-60A 35-70µm) oder C-18 RP-Kieselgel der Firma Macherey Nagel (Bezeichnung: Polygoprep 100-50 C18) eingesetzt.

Für die präparative Hochdruck Chromatographie werden Säulen der Firma Waters (Bezeichnung: XTerra Prep. MS C18, 5 µM, 30*100 mm oder Symmetrie C18, 5 µm, 19*100) verwendet.

### Nuclear Magnet Resonanz (NMR) Spektroskopie

Die Messung wird in deuteriertem Dimethylsulfoxid-d6 durchgeführt. Werden andere Lösungsmittel verwendet sind diese explizit in den Beispielen oder in den Methoden vermerkt. Die Messwerte werden auf einer Delta-Scala in der Einheit ppm angegeben. Als Standard wird Tetramethylsilan verwendet. Die Messung erfolgt auf einem Avance 400 (400MHz-NMR-Spektrometer) von der Firma Bruker Biospin GmbH.

### Massenspektroskopie / UV-Spektrometer

Diese Daten werden mit Hilfe einer HPLC-MS Anlage (high performance liquid chromatography mit Massendetektor) der Firma Agilent erzeugt. Die Anlage ist so aufgebaut, dass anschließend an die Chromatographie (Säule: Zorbax SB-C8, 3,5 µm, 2,1*50, Fa. Agilent) ein Diodenarry-Detektor (G1315B von Fa. Agilent) und ein Massendetektor (1100 LS-MSD SL; G1946D; Fa. Agilent) in Reihe geschalten sind.

Die Anlage wird mit einem Fluss von 1.2 mL/min betrieben. Für einen Trennvorgang wird ein Gradient innerhalb von 3,5 min durchlaufen (Gradient Anfang: 95 % Wasser und 5 % Acetonitril; Gradient Ende: 5 % Wasser und 95 % Acetonitril; beiden Lösungsmitteln wird jeweils 0.1 % Ameisensäure beigemischt).

### Beispiel 1

### 2-(4-Piperazin-1-yl-phenylamino)-4-((S)-4-oxo-2,3,10, 10a-tetrahydro-1H,4H-9-oxa-3a-aza-benzo[f]azulen-5-ylamino)-5-trifluormethyl-pyrimidin

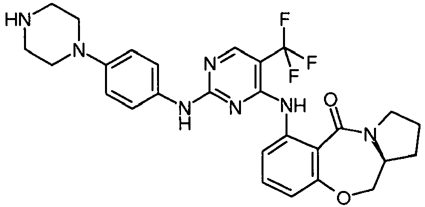

12.64 g (32,89 mmol) 4-(4-Amino-phenyl)-piperazin-1-carbonsäurebenzylester (Methode 3) werden in 10 mL Dioxan suspendiert mit 28 mL (164.4 mmol) Hünigbase versetzt und auf 0 °C abgekühlt. 5 g (23 mmol) 2,4-Dichlor-5-trifluorpyrimidin werden langsam zugegeben. Nachdem man 16 h bei Raumtemperatur gerührt hat, verdünnt man die Reaktionsmischung mit 200 mL Dichlormethan und 200 mL Natriumchloridlösung. Die organische Phase wird abgetrennt und das Lösungsmittel im Vakuum entfernt. Das so erhaltene Rohprodukt besteht aus beiden Regioisomeren. Diese werden durch Säulenchromatographie getrennt. Als Trägermaterial dient C18-RP-Kieselgel und es wird ein Gradient durchlaufen, der am Startpunkt aus 95 % Wasser und 5 % Acetonitril und am Endpunkt aus 5 % Wasser und 95 % Acetonitril besteht. Beiden Fließmitteln ist 0.2 % Ameisensäure beigemischt. Die Fraktionen, die 2-(4-Piperazin-1-yl-phenylamino)-4-chlor-5-trifluormethyl-pyrimidin enthalten, werden gefriergetrocknet.

400 mg (0.813 mmol) des so erhaltenen Zwischenprodukts und 185 mg (0,895 mmol) (S)-5-Amino-2,3,10,10a-tetrahydro-1*H*-9-oxa-3a-aza-benzo[f]azulen-4-on (Methode 1) werden in 0.5 ml N-Methyl-2-pyrrolidinon gelöst und mit 100 µL einer 4 M Lösung von HCl (0.406 mmol) in 1,4-Dioxan versetzt. Diese Mischung wird 1.5 h bei 100 °C gerührt. Dann gibt man 30 mL einer 1 N wässrigen Salzsäure zu und saugt den Niederschlag ab. Dieser wird getrocknet und mit 20 mL DMF und 1 mL Wasser gelöst mit 50 mg Palladium auf Kohle und 50 mg Palladiumhydroxid versetzt und 3 h bei 7 bar Wasserstoffdruck hydriert. Anschließend wird der Katalysator abfiltriert und das Lösungsmittel im Vakuum entfernt. Das Rohprodukt wird durch Säulenchromatographie gereinigt. Als Trägermaterial dient C18-RP-Kieselgel und es wird ein Gradient durchlaufen, der am Startpunkt aus 95 % Wasser und 5 % Acetonitril und am Endpunkt aus 45 % Wasser und 55 % Acetonitril besteht. Beiden Fließmitteln ist 0.2 % Ameisensäure beigemischt.

| | |
|---|---|
| Ausbeute: | 167 mg |
| UV max: | 282 nM |
| MS (ESI): | 540 (M+H) |
| ¹H-NMR: | 1,57-1,70 (m, 1H), 1,82-2,06 (m, 3H), 3,17-3,37 (m, 8H), 3,37-3,53 (m, 2H), 6,87-6,99 (m, 3H), 7,37-7,55 (m, 3H), 7,97-8,25 (m, 1H), 8,40 (s, 1H), 9,04-9,27 (m, 2H), 9,60-9,99 (m, 1H), 10,30-10,47 (m, 1H) |

### Beispiel 2

### 2-(4-(4-Methyl-piperazin-1-yl)-phenylamino)-4-((S)-4-oxo-2,3,10, 10a-tetrahydro-1H,4H-9-oxa-3a-aza-benzo[f]azulen-5-ylamino)-5-trifluormethyl-pyrimidin

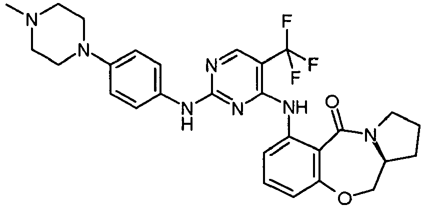

50 mg (0.077 mmol) 2-(4-Piperazin-1-yl-phenylamino)-4-((S)-4-oxo-2,3,10,10a-tetrahydro-1H,4H-9-oxa-3a-aza-benzo[f]azulen-5-ylamino)-5-trifluormethyl-pyrimidin werden in 0.5 mL Dimethylformamid gelöst und mit 12 µL (0.154 mmol) Formaldehyd und 2 µL (0.039 mmol) Essigsäure versetzt. Zu dieser Mischung dosiert man 86 mg (0.385 mmol) Natriumtriacetoxyborhydrid und rührt 16 h bei Raumtemperatur. Anschließend wird das Lösungsmittel im Vakuum entfernt. Das Rohprodukt wird durch Säulenchromatographie gereinigt. Als Trägermaterial dient C18-RP-Kieselgel und es wird ein Gradient durchlaufen, der am Startpunkt aus 95 % Wasser und 5 % Acetonitril und am Endpunkt aus 5 % Wasser und 95 % Acetonitril besteht. Beiden Fließmitteln ist 0.2 % Ameisensäure beigemischt. Die geeigneten Fraktionen werden mit 1 N wässriger Salzsäure versetzt und gefriergetrocknet.

| | |
|---|---|
| Ausbeute: | 49 mg |
| UV max: | 278 nM |
| MS (ESI): | 554 (M+H) |
| ¹H-NMR: | 1,58-1,72 (m, 1H), 1,84-2,10 (m, 3H), 2,87 (s, 3H), 2,95-3,08 (m, 2H), 3,12-3,26 (m, 2H), 3,38-3,48 (m, 1H), 3,49-3,59 (m, 2H), 3,63-3,73 (m, 1H), 3,75-3,89(m, 3H), 4,07-4,24 (m, 2H), 6,91-7,05 (m, 3H), 7,36-7,52 (m, 3H), 7,90-8,20 (s, 1H), 8,37-8,60 (s, 1H), 10,05-10,90 (m, 2H) |

### Beispiele 3-27

Die folgenden Verbindungen sind über ein analoges Verfahren, wie in Beispiel 1 beschrieben, hergestellt. Die entsprechenden Aniline sind in den Methoden 1, 2 oder 3 beschrieben oder sind kommerziell erhältlich.

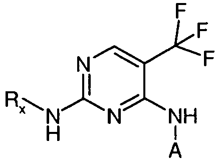

| # | A | Rₓ | UV max [nm] | MS (ESI) (M+H)⁺ |
|---|---|---|---|---|
| 3 | 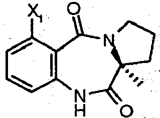 | 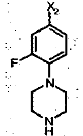 | 246,286 | 585 |
| 4 | 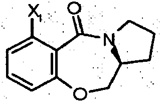 | 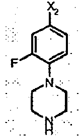 | 286 | 558 |
| 5 | 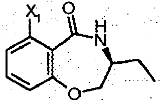 | 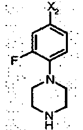 | 282 | 546 |
| 6 | 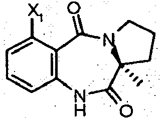 | 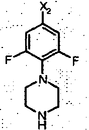 | 246,286 | 603 |
| 7 | 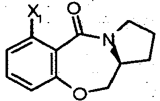 | 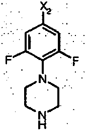 | 282 | 576 |
| 8 | 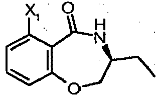 | 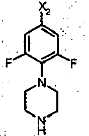 | 282 | 564 |
| 9 | 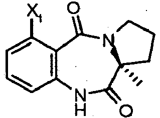 | 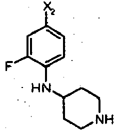 | 246,290 | 599 |
| 10 | 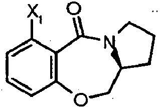 | 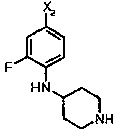 | 286 | 572 |
| 11 | 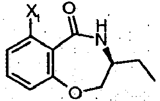 | 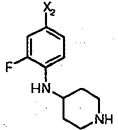 | 286,306 | 560 |
| 12 | 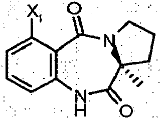 | 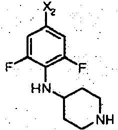 | 246,286 | 617 |
| 13 | 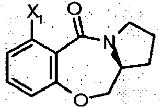 | 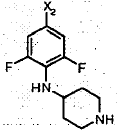 | 286 | 590 |
| 14 | 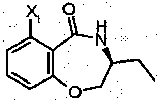 | 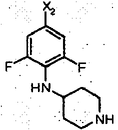 | 286 | 578 |
| 15 | 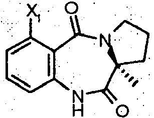 | 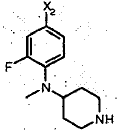 | 246,286 | 613 |
| 16 | 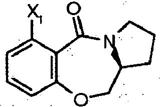 | 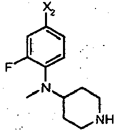 | 282 | 586 |
| 17 | 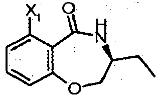 | 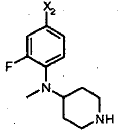 | 282 | 574 |
| 18 | 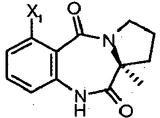 | 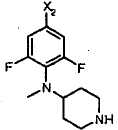 | 246,286 | 631 |
| 19 | 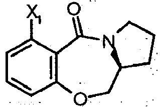 | 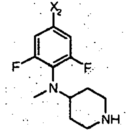 | 282 | 604 |
| 20 | 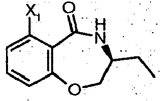 | 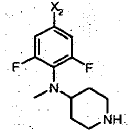 | 282 | 592 |
| 21 | 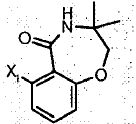 | 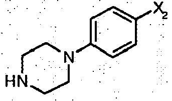 | 282-286 | 528 |
| 22 | 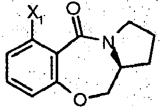 | 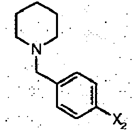 | 278 | 553 |
| 23 | 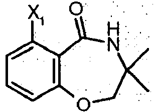 | 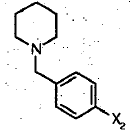 | 278-282 | 541 |
| 24 | 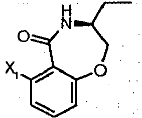 | 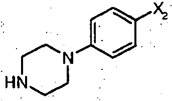 | 282 | 528 |
| 25 | 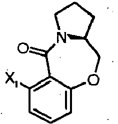 | 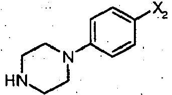 | 282 | 540 |
| 26 | 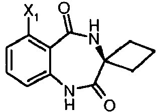 | 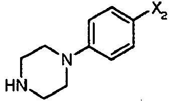 | 250,286 | 554 |
| 27 | 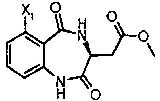 | 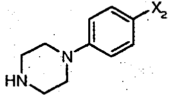 | 246,286 | 585 |

### Beispiele 28 - 81

Die folgenden Verbindungen sind über ein analoges Verfahren, wie in Beispiel 2 beschrieben, hergestellt. Die Herstellung des entsprechenden Ausgangsmaterials erfolgt über ein analoges Verfahren wie in Beispiel 1 beschrieben ist.

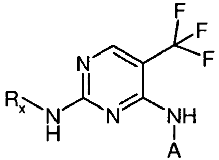

| # | A | Rₓ | UV max [nm] | MS (ESI) (M+H)⁺ |
|---|---|---|---|---|
| 28 | 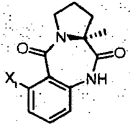 | 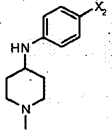 | 254,286 | 595 |
| 29 | 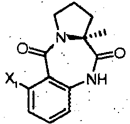 | 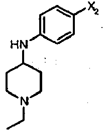 | 250,286 | 609 |
| 30 | 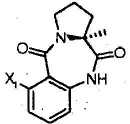 | 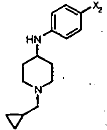 | 246,290 | 634 |
| 31 | 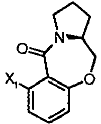 | 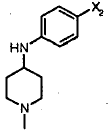 | 250,282 | 568 |
| 32 | 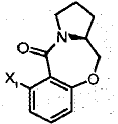 | 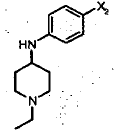 | 282 | 582 |
| 33 | 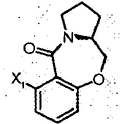 | 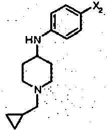 | 280,302 | 608 |
| 34 | 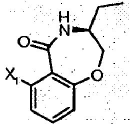 | 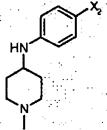 | 283 | 556 |
| 35 | 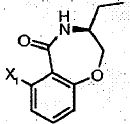 | 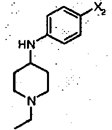 | 250,280 | 570 |
| 36 | 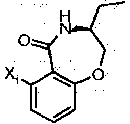 | 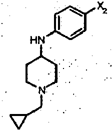 | 242,285, 306 | 596 |
| 37 | 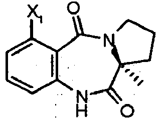 | 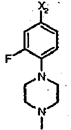 | 246,286 | 599 |
| 38 | 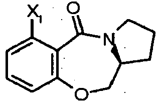 | 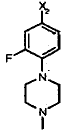 | 282 | 572 |
| 39 | 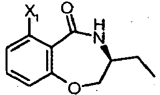 | 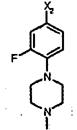 | 282 | 560 |
| 40 | 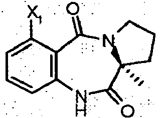 | 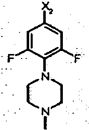 | 246,286 | 616 |
| 41 | 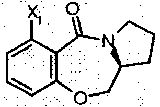 | 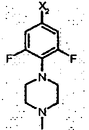 | 282 | 590 |
| 42 | 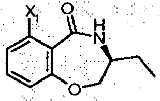 | 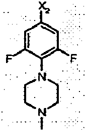 | 282 | 578 |
| 43 | 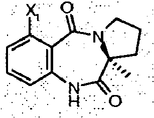 | 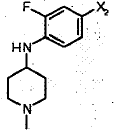 | 246,286 | 613 |
| 44 | 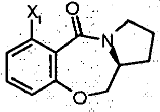 | 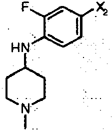 | 282 | 586 |
| 45 | 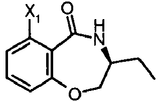 | 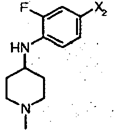 | 282 | 574 |
| 46 | 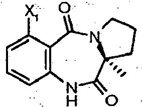 | 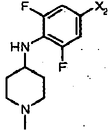 | 246,286 | 631 |
| 47 | 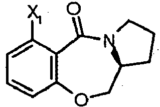 | 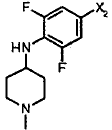 | 282 | 604 |
| 48 | 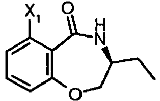 | 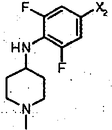 | 282 | 592 |
| 49 | 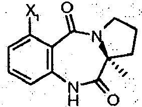 | 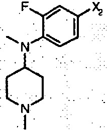 | 246,286 | 627 |
| 50 | 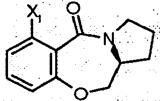 | 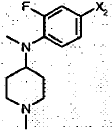 | 282 | 600 |
| 51 | 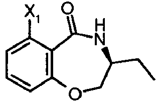 | 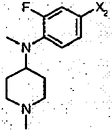 | 282 | 588 |
| 52 | 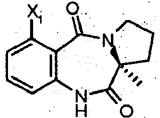 | 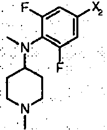 | 246,286 | 645 |
| 53 | 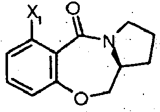 | 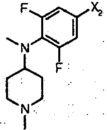 | 282 | 618 |
| 54 | 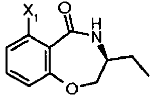 | 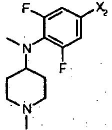 | 282 | 606 |
| 55 | 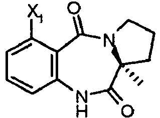 | 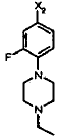 | 246,286 | 613 |
| 56 | 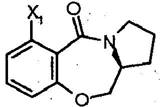 | 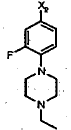 | 282 | 586 |
| 57 | 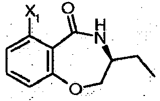 | 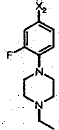 | 282 | 574 |
| 58 | 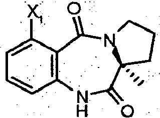 | 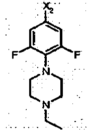 | 246,286 | 631 |
| 59 | 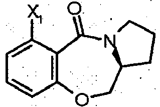 | 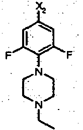 | 282 | 604 |
| 60 | 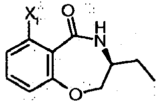 | 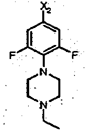 | 282 | 592 |
| 61 | 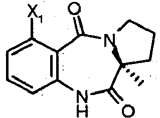 | 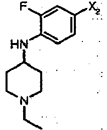 | 246,286 | 627 |
| 62 | 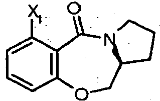 | 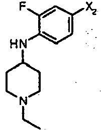 | 282 | 600 |
| 63 | 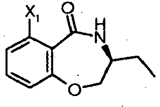 | 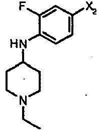 | 282 | 588 |
| 64 | 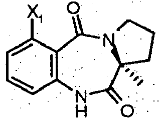 | 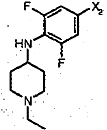 | 246,286 | 645 |
| 65 | 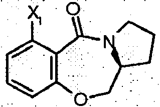 | 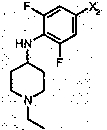 | 282 | 618 |
| 66 | 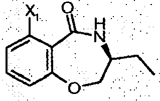 | 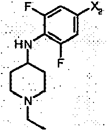 | 282 | 606 |
| 67 | 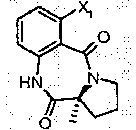 | 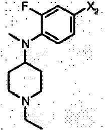 | 246,286 | 641 |
| 68 | 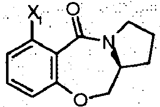 | 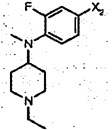 | 282 | 614 |
| 69 | 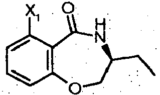 | 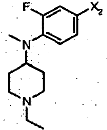 | 282 | 602 |
| 70 | 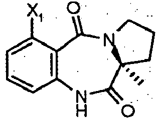 | 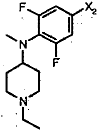 | 246,286 | 659 |
| 71 | 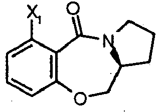 | 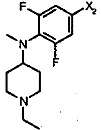 | 282 | 632 |
| 72 | 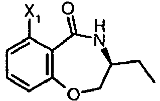 | 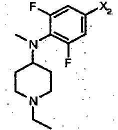 | 282 | 620 |
| 73 | 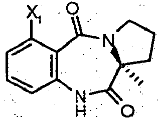 | 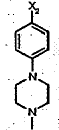 | 246,282 | 581 |
| 74 | 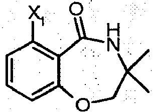 | 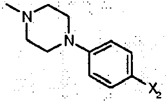 | 282-286 | 642 |
| 75 | 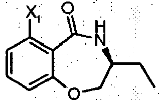 | 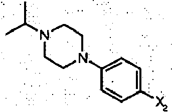 | 282 | 570 |
| 76 | 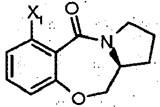 | 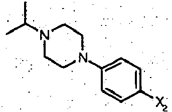 | 282-286 | 582 |
| 77 | 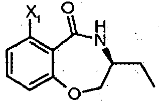 | 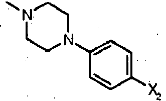 | 282 | 542 |
| 78 | 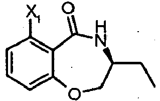 | 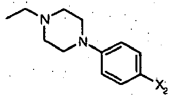 | 282 | 556 |
| 79 | 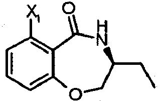 | 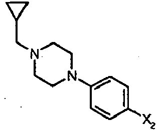 | 282 | 582 |
| 80 | 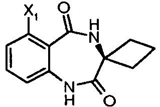 | 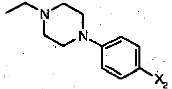 | 250,286 | 581 |
| 81 | 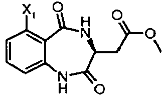 | 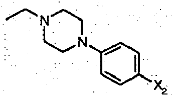 | 248,286 | 613 |

### Beispiele 82 - 91

Die folgenden Verbindungen sind über ein analoges Verfahren, wie in Beispiel 2 beschrieben, hergestellt. Die Herstellung des entsprechenden Ausgangsmaterials erfolgt über ein analoges Verfahren wie in Beispiel 1 beschrieben ist.

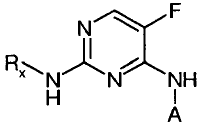

| # | A | Rₓ | UV max [nm] | MS (ESI) (M+H)⁺ |
|---|---|---|---|---|
| 82 | 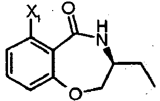 | 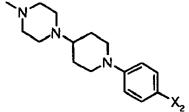 | 318 | 575 |
| 83 | 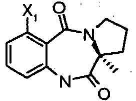 | 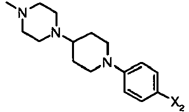 | 250-254 | 614 |
| 84 | 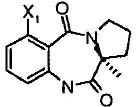 | 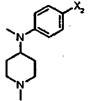 | 240/284 | 559 |
| 85 | 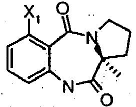 | 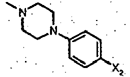 | 238/282/338 | 531 |
| 86 | 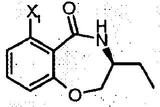 | 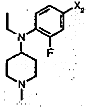 | 274 | 552 |
| 87 | 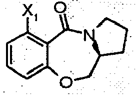 | 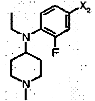 | 274 | 564 |
| 88 | 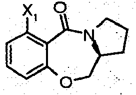 | 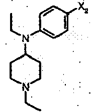 | 282 | 560 |
| 89 | 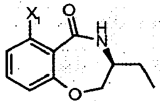 | 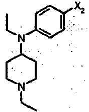 | 282 | 548 |
| 90 | 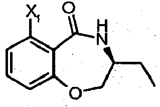 | 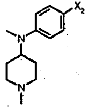 | 282 | 520 |
| 91 | 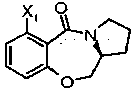 | 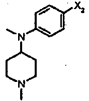 | 282 | 532 |

Die nachfolgenden Beispiele beschreiben die biologische Wirkung der erfindungsgemäßen Verbindungen ohne die Erfindung auf diese Beispiele einzuschränken.

Die Wirkung der erfindungsgemäßen Verbindungen auf verschiedene Kinasen, beispielsweise auf den Serin-Threonin Kinase PLK-1 und Aurora B, wird in in vitro Kinase Assays mit rekombinant hergestelltem Protein bestimmt. Die Verbindungen zeigen in diesen Assays eine gute bis sehr gute Wirksamkeit auf beiden Kinasen, d.h. beispielsweise einen IC50-Wert kleiner 1 µmol/L, in der Regel kleiner 0.1 µmol/L

### Beispiel PLK-1 Kinaseassay

Rekombinantes humanes und an seinem N-terminalen Ende mit GST verbundenes PLK1 Enzym wird aus Bakulovirus infizierten Insektenzellen (Sf21) isoliert. Die Reinigung erfolgt durch Affinitätschromatographie an Glutathion Sepharose Säulen.

4x10⁷ Sf21 Zellen (*Spodoptera frugiperda*) in 200 mL Sf-900 II Serum freien Insektenzellmedium (Life Technologies) werden in eine Spinnerflasche ausgesät. Nach 72 Stunden Inkubation bei 27 °C und 70 rpm werden 1x10⁸ Sf21 Zellen in insgesamt 180 mL Medium in eine neue Spinnerflasche ausgesät. Nach weiteren 24 Stunden werden 20 mL rekombinanter Baculovirus Stammsuspension zugesetzt und die Zellen 72 Stunden bei 27 °C bei 70 rpm kultiviert. 3 Stunden vor dem Ernten wird Okadainsäure zugesetzt (Calbiochem, Endkonzentration 0,1 µM) und die Suspension weiter inkubiert. Die Zellzahl wird bestimmt, die Zellen abzentrifugiert (5 Minuten, 4 °C, 800 rpm) und 1x mit PBS (8 g NaCl/L, 0.2 g KCl/L, 1.44 g Na₂HPO₄/L, 0.24 g KH₂PO4/L) gewaschen. Nach nochmaligem Abzentrifugieren wird das Pellet in flüssigem Stickstoff Schock gefroren. Danach wird das Pellet rasch aufgetaut und in eiskaltem Lysispuffer (50 mM HEPES pH 7.5, 10 mM MgCl₂, 1 mM DTT, 5 µg/mL Leupeptin, 5 µg/mL Aprotinin, 100 µM NaF, 100 µM PMSF, 10 mM ß-Glycerolphosphat, 0.1 mM Na₃VO₄, 30 mM 4-Nitrophenylphosphate) zu 1x10⁸ Zellen/ 17.5 mL resuspendiert. Die Zellen werden 30 Minuten auf Eis lysiert. Nach dem Entfernen der Zelltrümmer durch Zentrifugation (4000 rpm, 5 Minuten) wird der klare Überstand mit Glutathion Sepharosebeads versetzt (1 mL resuspendierte und gewaschene Beads für 50 mL Überstand) und 30 Minuten bei 4 °C auf einem Rotationsbrett inkubiert. Danach werden die Beads mit Lysispuffer gewaschen und das rekombinante Protein mit 1 mL Elutionspuffer/mL resuspendierte Beads (Elutionspuffer: 100 mM Tris/HCl pH=8, 120 mM NaCl, 20 mM reduziertes Glutathion (Sigma G-4251), 10 mM MgCl₂, 1 mM DTT) von den Beads eluiert. Die Proteinkonzentration wird mittels Bradford Assay bestimmt.

### KinaseAssay

In einem Napf einer 96-Loch Rundbodenplatte (Fa. Greiner bio-one, PS-Microtiterplatte Nr.650101) werden folgende Komponenten zusammengefügt:
- 10 µL zu testende Verbindung in variabler Konzentration (z.B. beginnend bei 300 µM, und Verdünnung in 1:3) in 6 % DMSO, 0.5 mg/mL Casein (Sigma C-5890), 60 mM ß-Glycerophosphat, 25 mM MOPS pH=7,0, 5 mM EGTA, 15 mM MgCl₂, 1 mM DTT
- 20 µL Substratlösung (25 mM MOPS pH=7, 15 mM MgCl₂, 1 mM DTT, 2,5 mM EGTA, 30 mM ß-Glycerophosphat, 0.25 mg/mL Casein)
- 20 µL Enzymverdünnung (1:100 Verdünnung des Enzymstocks in 25 mM MOPS pH=7, 15 mM MgCl₂, 1 mM DTT)
- 10 µL ATP Lösung (45 µM ATP mit 1,11x10⁶ Bq/ml gamma-P33-ATP).

Durch Zusatz der ATP Lösung wird die Reaktion gestartet und 45 Minuten bei 30 °C unter leichtem Schütteln (650 rpm auf IKA Schüttler MTS2) durchgeführt. Die Reaktion wird durch Zusatz von 125 µL eiskalter 5 %iger TCA pro Napf gestoppt und mindestens 30 Minuten auf Eis inkubiert. Das Präzipitat wird durch Ernten auf Filterplatten (96-well-Microtiter-Filterplatte: UniFilter-96, GF/B; Fa. Packard; Nr.6005177) übertragen, dann 4 mal mit 1 %iger TCA gewaschen und bei 60 °C getrocknet. Nach Zugabe von 35 µL Szintillationslösung (Ready-Safe; Beckmann) pro Napf wird die Platte mit Sealing-tape zugeklebt und die präzipitierte Menge P33 mit dem Wallac Betacounter gemessen.

Die Messdaten werden mit der Standard Graphpad Software (Levenburg-Marquard Algorhythmus) ausgewertet.

### Beispiel Aurora-B Kinaseassay

Es wurde ein radioaktiver Enzymhemmungs-Assay entwickelt, der Baculovirusexprimiertes rekombinantes N-terminal mit einem Histidine(6)-Epitop (His-) ausgestattetes humanes Aurora B Wildtyp Protein verwendet, das aus infizierten Insekten Zellen (SF21) gewonnen und gereinigt wird.

### Expression und Reinigung

Hierzu werden beispielsweise 300x10⁶ SF21 Zellen in SF-900II Insektenzell-Medium (Invitrogen) mit einer passenden Menge Bakuloviruslösung für 1 h bei 27 °C inkubiert (Fernbachkolben-Schüttler, 50 rpm). Danach werden 250 mL SF-900 II Medium zugegeben und für 3 Tage geschüttelt (100 rpm, 27 °C). 3 h vor der Ernte wird der Kultur Okadainsäure (C₄₄H₆₈O₁₃, Calbiochem #495604) zugesetzt (Endkonzentration 0.1 µM) um Phosphorylierungsstellen auf rekombinantem Aurora B zu stabilisieren. Die Zellen werden durch Zentrifugation (1000 rpm, 5 min, 4 °C) pelletiert, der Überstand verworfen und das Pellet in flüssigem Stickstoff gefroren. Das Pellet wird aufgetaut (37 °C, 5 min) und in Lysispuffer resuspendiert. Für 200 mL Ausgangskulturvolumen werden 40 mL Lysispuffer (25 mM Tris/Cl, 10 mM MgCl₂, 300 mM NaCl, 20 mM Imidazol, pH 8, 0.07 % 2-Mercaptoethanol und Protease-Inhibitor-Complete von Roche Diagnostics) verwendet. Nach 2 zügigen Gefrier/Auftauzyklen (Flüssigstickstoff zu 37 °C), wird das Lysat für 30 min auf Eis gehalten, danach mit gewaschenen Ni-NTA Kügelchen (Ni-NTA Superflow Beads, 4 mL pro 200 mL Ausgangskultur) inkubiert (2 h, 4 °C) und in eine Econo-Pac Säule (Biorad #732-1010) gefüllt. 5 Waschungen mit jeweils 10 Säulenvolumen Waschpuffer (25 mM Tris/Cl, 10 mM MgCl₂, 1000 mM NaCl, 20 mM Imidazol, pH 8, 0.07 % 2-Mercaptoethanol und Protease-Inhibitor-Complete von Roche Diagnostics) gehen der Elution in 8mL (pro 200 mL Ausgangskultur) Elutionspuffer (25 mM Tris/Cl pH 8, 300 mM NaCl, 10 mM MgCl2, 0.03 % Brij-35, 10 % Glycerol , 0.07% 2-Mercaptoethanol, 400 mM Imidazol) voraus. Die vereinigten Eluatfraktionen werden mittels einer Sephadex G25-Säule entsalzt und in Einfrierpuffer (50 mM Tris/Cl pH 8, 150 mM NaCl, 0.1 mM EDTA, 0.03% Brij-35, 10% Glycerol, 1 mM DTT) überführt.

### Kinaseassay

Testsubstanzen werden in eine Polypropylenplatte (96 Vertiefungen, Greiner #655 201) vorgelegt, um einen Konzentrationsrahmen von 10 µM - 0.0001 µM abzudecken. Die Endkonzentration von DMSO im Assay ist 5 %. Zu vorgelegten 10 µL Testsubstanz in 25 % DMSO werden 30 µL Protein-Mix (50 mM Tris/Cl pH 7.5,25 mM MgCl₂, 25 mM NaCl, 167 µM ATP, 200 ng His-Aurora B in Einfrierpuffer) pipettiert und 15 min bei RT inkubiert. Danach werden 10 µL Peptid-Mix (100 mM Tris/Cl pH 7.5, 50 mM MgCl₂, 50 mM NaCl, 5 µM NaF, 5 µM DTT, 1 µCi gamma-P33-ATP [Amersham], 50 µM Substratpeptid [Biotin-EPLERRLSLVPDS oder Multimere davon, oder Biotin-EPLERRLSLVPKM oder Multimere davon, bzw. Biotin-LRRWSLGLRRWSLGLRRWSLGLRRWSLG]) zugegeben. Die Reaktion wird 75 min inkubiert (RT) und durch Zugabe von 180 µL 6.4 % Trichloressigsäure gestoppt und für 20 min auf Eis inkubiert. Eine Multiscreen Filtrationsplatte (Millipore, MAIP NOB10) wird mit zunächst 100 µL 70 % Ethanol und danach mit 180 µL Trichloressigsäure äquilibriert und die Flüssigkeiten mit einem geeigneten Absauggerät entfernt. Danach wird die gestoppte Kinasereaktion aufgebracht. Nach 5 Waschschritten mit jeweils 180 µL 1 % Trichloressigsäure wird der untere Teil der Platte getrocknet (10-20 min bei 55 °C) und 25 µL Scintillations-Cocktail (Microscint, Packard # 6013611) zugegeben. Inkorporiertes gamma-Phosphat wird mit Hilfe eines Wallac 1450 Microbeta Flüssigscintillations Zählers quantifiziert. Proben ohne Testsubstanz oder ohne Substratpeptid dienen als Kontrollen. IC₅₀ Werte werden mittels Graph Pad Prism Software ermittelt.

Die antiproliferative Wirkung der erfindungsgemäßen Verbindungen wird im Cytotoxizitätstest an kultivierten humanen Tumorzellen und/oder in einer FACS-Analyse, beispielsweise an HeLa S3-Zellen, bestimmt. Die Verbindungen zeigen in beiden Testmethoden eine gute bis sehr gute Wirksamkeit, d.h. beispielsweise einen EC50-Wert im HeLa S3-Cytotoxizitätstest kleiner 5 µmol/L, in der Regel kleiner 1 µmol/L.

### Messung der Cytotoxizität an kultivierten humanen Tumorzellen

Zur Messung der Cytotoxizität an kultivierten humanen Tumorzellen werden Zellen der zervikalen Carcinoma Tumorzell-Linie HeLa S3 (erhalten von American Type Culture Collection (ATCC)) in Ham's F12 Medium (Life Technologies) und 10 % fötalem Rinderserum (Life Technologies) kultiviert und in der log-Wachstumsphase geerntet. Anschließend werden die HeLa S3 Zellen in 96-well Platten (Costar) mit einer Dichte von 1000 Zellen pro well eingebracht und über Nacht in einem Inkubator (bei 37 °C und 5 % CO₂) inkubiert, wobei auf jeder Platte 6 wells nur mit Medium gefüllt werden (3 wells zur Mediumkontrolle, 3 wells zur Inkubation mit reduzierten AlamarBlue Reagenz). Die Wirksubstanzen werden in verschiedenen Konzentrationen (gelöst in DMSO; DMSO-Endkonzentration: 0.1 %) zu den Zellen zugegeben (jeweils als Dreifachbestimmung). Nach 72 Stunden Inkubation werden zu jedem well 20 µL AlamarBlue Reagenz (AccuMed International) zugesetzt, und die Zellen für weitere 5-7 Stunden inkubiert. Zur Kontrolle wird zu 3 wells je 20 µL reduziertes AlamarBlue Reagenz gegeben (AlamarBlue Reagenz, das für 30 min autoklaviert wird). Nach Inkubation wird der Farbumsatz des AlamarBlue Reagenz in den einzelnen wells in einem Perkin Elmer Fluoreszenzspektrophotometer bestimmt (Exitation 530 nm, Emission 590 nm, Slits 15, Integrate time 0.1). Die Menge an umgesetzten AlamarBlue Reagenz repräsentiert die metabolische Aktivität der Zellen. Die relative Zellaktivität wird in Prozent der Kontrolle (HeLa S3 Zellen ohne Inhibitor) berechnet und die Wirkstoffkonzentration, die die Zellaktivität zu 50 % hemmt (IC50) abgeleitet. Die Werte werden hierbei aus dem Mittelwert von drei Einzelbestimmungen - unter Korrektur des Leerwertes (Mediumkontrolle) - berechnet.

### FACS-Analyse

Propidiumjodid (PI) bindet stöchiometrisch an doppelsträngige DNA, und ist damit geeignet den Anteil an Zellen in der G1, S, und G2/M Phase des Zellzykluses auf der Basis des zellulären DNA Gehaltes zu bestimmen. Zellen in der G0 und G 1 Phase haben einen diploiden DNA Gehalt (2N), während Zellen in der G2 oder Mitose einen 4N DNA Gehalt haben.

Für eine PI-Färbung werden beispielsweise 1x10⁶ HeLa S3 Zellen auf eine 75 cm2 Zellkulturflasche ausgesät, nach 24 h wird entweder 0.1 % DMSO als Kontrolle zugesetzt, bzw. die Substanz in verschiedenen Konzentrationen (in 0.1 % DMSO). Die Zellen werden für 24 h mit der Substanz bzw. mit DMSO inkubiert, bevor die Zellen 2 mal mit PBS gewaschen und dann mit Trypsin /EDTA abgelöst werden. Die Zellen werden zentrifugiert (1000 Upm, 5 min, 4 °C), und das Zellpellet 2 mal mit PBS gewaschen, bevor die Zellen in 0.1 mL PBS resuspendiert werden. Anschließend werden die Zellen für 16 Stunden bei 4 °C oder alternativ für 2 Stunden bei -20 °C mit 80 % Ethanol fixiert. Die fixierten Zellen werden zentrifugiert (1000 Upm, 5 min, 4 °C), mit PBS gewaschen und anschließend nochmals zentrifugiert. Das Zellpellet wird in 2 mL 0.25 % Triton X-100 in PBS resuspendiert, und 5 min auf Eis inkubiert, bevor 5 mL PBS zugeben werden und erneut zentrifugiert wird. Das Zellpellet wird in 350 µL PI Färbelösung (0.1 mg/mL RNase A (Sigma, No. R-4875), 10 µg/mL Propidiumjodid (Sigma, No. P-4864) in 1 x PBS) resuspendiert. Die Zellen werden für 20 min im Dunkeln mit dem Färbepuffer inkubiert, bevor sie in Probenmessgefäße für das FACS Scan überführt werden. Die DNA Messung erfolgt in einem Becton Dickinson FACS Analyzer, mit einem Argonlaser (500 mW, Emission 488 nm), und dem DNA Cell Quest Programm (BD). Die logarithmische PI Fluoreszenz wird mit einem band-pass Filter (BP 585/42) bestimmt. Die Quantifizierung der Zellpopulationen in den einzelnen Zellzyklusphasen erfolgt mit dem ModFit LT Programm von Becton Dickinson.

Entsprechend werden die erfindungsgemäßen Verbindungen auch auf weiteren Tumorzellen getestet. Beispielsweise sind diese Verbindungen auf Karzinomen verschiedenster Gewebe (z. Bsp. Brust (MCF7); Colon (HCT116), Kopf-Hals (FaDu), Lunge (NCI-H460), Pankreas (BxPC-3), Prostata (DU145)), Sarkome (z. Bsp. SK-UT-1B), Leukämien und Lymphome (z. Bsp. HL-60; Jurkat, THP-1) und anderen Tumoren (z. Bsp. Melanome (BRO), Gliome (U-87MG)) aktiv und könnten in solchen Indikationen eingesetzt werden. Dies belegt die breite Anwendbarkeit der erfindungsgemäßen Verbindungen zur Behandlung verschiedenster Tumortypen.

Auf Grund ihrer biologischen Eigenschaften eignen sich die neuen Verbindungen der allgemeinen Formel (1), deren Isomere und deren physiologisch verträgliche Salze zur Behandlung von Erkrankungen, die durch exzessive oder anomale Zellproliferation charakterisiert sind.

Zu solchen Erkrankungen gehören beispielsweise: Virale Infektionen (z.B. HIV und Kaposi Sarkoma), Entzündung und Autoimmunerkrankungen (z.B. Colitis, Arthritis, Alzheimer Erkrankung, Glomerulonephritis und Wundheilung), bakterielle, fungale und/oder parasitäre Infektionen, Leukämien, Lymphoma und solide Tumore (zB. Karzinome und Sarkome), Hauterkrankungen (z.B. Psoriasis), auf Hyperplasie beruhende Krankheiten die durch einen Anstieg der Zellzahl (z.B. Fibroblasten, Hepatozyten, Knochen und Knochenmarkzellen, Knorpel oder glatte Muskulaturzellen oder Epithelialzellen (z.B. endometrische Hyperplasie)) gekennzeichnet sind; Knochenerkrankungen und kardiovaskuläre Erkrankungen (z.B. Restenose und Hypertrophie).

Beispielsweise können folgende Krebserkrankungen mit erfindungsgemäßen Verbindungen behandelt werden, ohne jedoch darauf beschränkt zu sein: Hirntumore wie beispielsweise Akustikusneurinom, Astrozytome wie pilozytisches Astrozytome, fibrilläres Astrozytom, protoplasmatisches Astrozytom, gemistozytäres Astrozytom, anaplastisches Astrozytom und Glioblastome, Hirnlymphome, Hirnmetastasen, Hypophysentumor wie Prolaktinom, HGH (human growth hormon) produzierender Tumor und ACTH produzierender Tumor (adrenocorticotropes Hormon), Kraniopharyngiome, Medulloblastome, Meningeome und Oligodendrogliome; Nerventumore (Neoplasmen) wie zum Beispiel Tumore des vegetativen Nervensystems wie Neuroblastoma sympathicum, Ganglioneurom, Paragangliom (Phäochromozytom, Chromaffinom) und Glomuscaroticum-Tumor, Tumore am peripheren Nervensystem wie Amputationsneurom, Neurofibrom, Neurinom (Neurilemmom, Schwannom) und malignes Schwannom, sowie Tumore am zentralen Nervensystem als Hirn- und Rückenmarkstumoren; Darmkrebs wie zum Beispiel Rektumkarzinom, Kolonkarzinom, Analkarzinom, Dünndarmtumore und Zwölffingerdarmtumore; Lidtumore wie Basaliom oder Basalzellkarzinom; Bauchspeicheldrüsenkrebs oder Pankreaskarzinom; Blasenkrebs oder Blasenkarzinom; Lungenkrebs (Bronchialkarzinom) wie beispielsweise kleinzellige Bronchialkarzinome (Haferzellkarzinome) und nicht-kleinzellige Bronchialkarzinome wie Plattenepithelkarzinome, Adenokarzinome und großzellige Bronchialkarzinome; Brustkrebs wie zum Beispiel Mammakarzinom wie infiltrierend duktales Karzinom, Kolloidkarzinom, lobulär invasives Karzinom, tubuläres Karzinom, adenozystisches Karzinom und papilläres Karzinom; Non-Hodgkin-Lymphomen (NHL) wie beispielsweise Burkitt-Lymphom, niedrigmaligne Non-Hodgkin-Lymphomen (NHL) und Mucosis fungoides; Gebärmutterkrebs oder Endometriumkarzinom bzw. Corpuskarzinom; CUP-Syndrom (Cancer of Unknown Primary); Eierstockskrebs oder Ovarial-Karzinom wie mucinöse, endometriale oder seröse Krebs; Gallenblasenkrebs; Gallengangskrebs wie beispielsweise Klatskin-Tumor; Hodenkrebs wie zum Beispiel Seminome und Nicht-Seminome; Lymphom (Lymphosarkom) wie zum Beispiel malignes Lymphom, Morbus Hodgkin, Non-Hodgkin-Lymphomen (NHL) wie chronisch lymphatische Leukämie, Haarzell-Leukämie, Immunozytom, Plasmozytom (multiples Myelom), Immunoblastom, Burkitt-Lymphom, T-Zonen-Mycosis fungoides, großzellig-anaplastisches Lymphoblastom und Lymphoblastom; Kehlkopfkrebs wie zum Beispiel Stimmbandtumoren, supraglottisch, glottisch und subglottisch Kehlkopftumore; Knochenkrebs wie zum Beispiel Osteochondrom, Chondrom, Chondroblastom, Chondromyxoidfibrom, Osteom, Osteoid-Osteom, Osteoblastom, eosinophiles Granulom, Riesenzell-Tumor, Chondrosarkom, Osteosarkom, Ewing-Sarkom, Retikulo-Sarkom, Plasmozytom, Riesenzell-Tumor, fibröse Dysplasie, juvenile Knochenzyste und aneurysmatische Knochenzyste; Kopf-Hals-Tumoren wie zum Beispiel Tumoren der Lippen, Zunge, Mundboden, Mundhöhle, Zahnfleisch, Gaumen, Speicheldrüsen, Rachen, Nasenhöhlen, Nasennebenhöhlen, Kehlkopf und Mittelohr; Leberkrebs wie zum Beispiel das Leberzellkarzinom oder hepatozelluläres Karzinom (HCC); Leukämien wie zum Beispiel akute Leukämien wie akute lymphatische/lymphoblastische Leukämie (ALL), akute myeloische Leukämie (AML); chronische Leukämien wie chronisch lymphatische Leukämie (CLL), chronisch myeloische Leukämie (CML); Magenkrebs oder Magenkarzinom wie zum Beispiel papilläres, tubuläres und muzinöses Adenokarzinom, Siegelringzellkarzinom adenosquamöses Karzinom, kleinzelliges Karzinom und undifferenziertes Karzinom; Melanome wie zum Beispiel oberflächlich spreitendes, knotiges, lenitgo-maligna und akral-lentiginöse Melanom; Nierenkrebs wie zum Beispiel Nierenzellkarzinom oder Hypernephrom oder Grawitz-Tumor; Speiseröhrenkrebs oder Ösophaguskarzinom; Peniskrebs; Prostatakrebs; Rachenkrebs oder Pharynxkarzinome wie zum Beispiel Nasopharynxkarzinome, Oropharynxkarzinome und Hypopharynxkarzinome; Retinoblastom; Scheidenkrebs oder Vaginalkarzinom; Plattenepithelkarzinome, Adenokarzinome, in situ Karzinome, maligne Melanome und Sarkome; Schilddrüsen-Karzinome wie zum Beispiel papilläre, follikuläre und medulläre Schilddrüsen-Karzinom, sowie anaplastische Karzinome; Spinaliom, Stachelzellkarzinom und Plattenepithelkarzinom der Haut; Thymome, Urethrakrebs und Vulvakrebs.

Die neuen Verbindungen können zur Prävention, Kurz- oder Langzeitbehandlung der vorstehend erwähnten Krankheiten gegebenenfalls auch in Kombination mit Radiotherapie oder mit anderen "State-of-art" Verbindungen, wie z.B. zytostatische oder zytotoxische Substanzen, Zellproliferationshemmer, anti-angiogene Substanzen, Steroide oder Antikörper, verwendet werden.

Die Verbindungen der allgemeinen Formel (1) können allein oder in Kombination mit anderen erfindungsgemäßen Wirkstoffen, gegebenenfalls auch in Kombination mit weiteren pharmakologisch aktiven Wirkstoffen, zur Anwendung gelangen.

Chemotherapeutica welche in Kombination mit den erfindungsgemäßen Verbindungen verabreicht werden können, umfassen ohne darauf eingeschränkt zu sein, Hormone, Hormonanaloge und Antihormone (z.B. Tamoxifen, Toremifen, Raloxifen, Fulvestrant, Megestrol Acetat, Flutamid, Nilutamid, Bicalutamid, Aminoglutethimid, Cyproteron Acetat, Finasterid, Buserelin Acetat, Fludrocortinson, Fluoxymesteron, Medroxyprogesteron, Octreotid), Aromatase Inhibitoren (z.B. Anastrozol, Letrozol, Liarozol, Vorozol, Exemestan, Atamestan,), LHRH Agonisten und Antagonisten (z.B. Goserelin Acetat, Luprolid), Inhibitoren von Wachstumsfaktoren (growth factors wie zum Beispiel "platelet derived growth factor" und "hepatocyte growth factor", Inhibitoren sind z. B. "growth factor"-Antikörper, "growth factor receptor"-Antikörper und Tyrosinekinase Inhibitoren, wie zum Beispiel Gefitinib, Imatinib, Lapatinib und Trastuzumab); Antimetabolite (z.B. Antifolate wie Methotrexat, Raltitrexed, Pyrimidinanaloge wie 5-Fluorouracil, Capecitabin und Gemcitabin, Purin- und Adenosinanaloge wie Mercaptopurin, Thioguanin, Cladribin und Pentostatin, Cytarabin, Fludarabin); Antitumor-Antibiotica (z.B. Anthracycline wie Doxorubicin, Daunorubicin, Epirubicin und Idarubicin, Mitomycin-C, Bleomycin, Dactinomycin, Plicamycin, Streptozocin); Platinderivative (z.B. Cisplatin, Oxaliplatin, Carboplatin); Alkylierungsagentien (z.B. Estramustin, Meclorethamin, Melphalan, Chlorambucil, Busulphan, Dacarbazin, Cyclophosphamid, Ifosfamid, Temozolomid, Nitrosoharnstoffe wie zum Beispiel Carmustin und Lomustin, Thiotepa); antimitotische Agentien (z.B. Vinca-Alkaloide wie zum Beispiel Vinblastin, Vindesin, Vinorelbin und Vincristin; und Taxane wie Paclitaxel, Docetaxel); Topoisomerase Inhibitoren (z.B. Epipodophyllotoxine wie zum Beispiel Etoposid und Etopophos, Teniposid, Amsacrin, Topotecan, Irinotecan, Mitoxantron) und verschiedene Chemotherapeutica wie Amifostin, Anagrelid, Clodronat, Filgrastin, Interferon alpha, Leucovorin, Rituximab, Procarbazin, Levamisol, Mesna, Mitotan, Pamidronat und Porfimer.

Geeignete Anwendungsformen sind beispielsweise Tabletten, Kapseln, Zäpfchen, Lösungen, - insbesondere Lösungen zur Injektion (s.c., i.v., i.m.) und Infusion - Säfte, Emulsionen oder dispersible Pulver. Hierbei soll der Anteil der pharmazeutisch wirksamen Verbindung(en) jeweils im Bereich von 0.1 - 90 Gew.-%, bevorzugt 0.5 - 50 Gew.-% der Gesamtzusammensetzung liegen, das heißt in Mengen die ausreichend sind, um den unten angegebenen Dosierungsbereich zu erreichen. Die genannten Dosen können, falls erforderlich, mehrmals täglich gegeben werden.

Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektions- und Infusionslösungen werden in üblicher Weise, z.B. unter Zusatz von Isotonantien, Konservierungsmitteln, wie p-Hydroxybenzoate, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilfslösemittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyethylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Die Applikation erfolgt in üblicher Weise, vorzugsweise oral oder transdermal, insbesondere bevorzugt oral. Im Falle der oralen Anwendung können die Tabletten selbstverständlich außer den genannten Trägerstoffen auch Zusätze, wie z.B. Natriumcitrat, Calciumcarbonat und Dikalziumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wässriger Suspensionen können die Wirkstoffe außer den oben genannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Die Dosierung für die intravenöse Anwendung liegt bei 1 - 1000 mg pro Stunde, vorzugsweise zwischen 5 - 500 mg pro Stunde.

Trotzdem kann es gegebenenfalls notwendig sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Formulierungsbeispiele illustrieren die vorliegende Erfindung ohne sie jedoch in ihrem Umfang zu beschränken.

### Pharmazeutische Formulierungsbeispiele

| A) | Tabletten | pro Tablette |
|---|---|---|
| | Wirkstoff nach Formel (1) | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der fein gemahlene Wirkstoff, Milchzucker und ein Teil der Maisstärke werden miteinander vermischt. Die Mischung wird gesiebt, danach mit einer Lösung von Polyvinylpyrrolidon in Wasser befeuchtet, geknetet, feucht granuliert und getrocknet. Das Granulat, der Rest der Maisstärke und das Magnesiumstearat werden gesiebt und miteinander vermischt. Das Gemisch wird zu Tabletten geeigneter Form und Größe verpresst.

| B) | Tabletten | pro Tablette |
|---|---|---|
| | Wirkstoff nach Formel (1) | 80 mg |
| | Milchzucker | 55 mg |
| | Maisstärke | 190 mg |
| | Mikrokristalline Cellulose | 35 mg |
| | Polyvinylpyrrolidon | 15 mg |
| | Natrium-carboxymethylstärke | 23 mg |
| | Magnesiumstearat | 2 mg |
| | | 400 mg |

Der fein gemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpresst das Gemisch zu Tabletten geeigneter Größe.

| C) | Ampullenlösung | |
|---|---|---|
| | Wirkstoff nach Formel (1) | 50 mg |
| | Natriumchlorid | 50 mg |
| | Aqua pro inj. | 5 ml |

Der Wirkstoff wird bei Eigen-pH oder gegebenenfalls bei pH 5.5 - 6.5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

## Patentansprüche

1. Verbindungen der allgemeinen Formel (1) 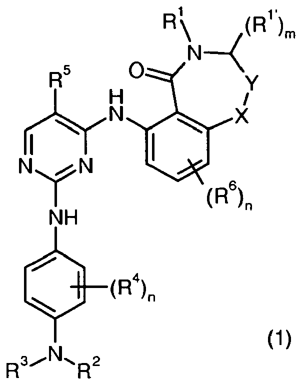 worin
X Sauerstoff oder Stickstoff, und
Y -CH₂-oder C=O, und
R¹ und R^{1'} jeweils unabhängig voneinander Wasserstoff oder ein gegebenenfalls einfach oder mehrfach substituierter Rest ausgewählt aus der Gruppe bestehend aus C₁₋₅Alkyl, C₃₋₆Cycloalkyl, Aryl, Heterocyclyl und Heteroaryl, wobei der/die Substituenten gleich oder verschieden sein können und ausgewählt werden aus der Gruppe bestehend aus Halogen, -OR^{a}, -C(=O)R^{a}, -C(=O)OR^{a}, -C(=O)NR^{a}R^{a}, -NR^{a}R^{a}, -NR^{a}C(=O)R^{a}, -NR^{a}C(=O)OR^{a}, -NR^{a}C(=O)NR^{a}R^{a}, -NR^{a}SO₂R^{a}, -N=CR^{a}R^{a}, -SR^{a}, -SOR^{a}, -SO₂R^{a}, -SO₂NR^{a}R^{a}, -NR^{a}SO₂NR^{a}R^{a}, -OSO₂NR^{a}R^{a} und Pseudohalogen, oder gegebenenfalls können die am gleichen C-Atom befindlichen R^{1'} miteinander einen C₃₋₇Cycloalkylring bilden,
oder gegebenenfalls kann R¹ mit einem R^{1'} eine gemeinsame gesättigte oder teilweise ungesättigte 3-6-gliedrigen Alkylbrücke, die gegebenenfalls ein bis zwei Heteroatome enthalten kann, verbunden sein, und
R² ein gegebenenfalls einfach oder mehrfach substituierter Heterocyclyl Rest, wobei der/die Substituenten gleich oder verschieden sein können und ausgewählt werden aus der Gruppe bestehend aus C₁₋₅Alkyl, C₂₋₅Alkenyl, C₂₋₅Alkinyl, C₃₋₆CycloalkylC₀₋₃Alkyl, und
R³ Wasserstoff oder C₁₋₃Alkyl, oder
R² und R³ gemeinsam einen gegebenenfalls einfach oder mehrfach substituierten Heterocyclylring bilden, der gegebenenfalls ein bis zwei weitere Heteroatome enthalten kann, wobei der/die Substituenten gleich oder verschieden sein können und ausgewählt werden aus der Gruppe bestehend aus C₁₋₅Alkyl, C₂₋₅Alkenyl, C₂₋₅Alkinyl und C₃₋₆CycloalkylC₀₋₃Alkyl, oder ein geeigneter Substituent ausgewählt aus der Gruppe bestehend aus Halogen, -OR^{a}, -C(=O)R^{a}, -C(=O)OR^{a}, -C(=O)NR^{a}R^{a}, -NR^{a}R^{a}, -NR^{a}C(=O)R^{a}, -NR^{a}C(=O)OR^{a}, -NR^{a}C(=O)NR^{a}R^{a}, -NR^{a}SO₂R^{a}, -N=CR^{a}R^{a}, -SR^{a}, -SOR^{a}, -SO₂R^{a}, -SO₂NR^{a}R^{a}, -NR^{a}SO₂NR^{a}R^{a}, -OSO₂NR^{a}R^{a} und Pseudohalogen, und
R⁴ jeweils unabhängig voneinander ein Rest ausgewählt aus der Gruppe bestehend aus Halogen, NO₂, -OR^{a}, -C(=O)R^{a}, -C(=O)OR^{a}, -C(=O)NR^{a}R^{a}, -NR^{a}R^{a}, -NR^{a}COR^{a}, -NR^{a}C(=O)OR^{a}, -NR^{a}C(=O)NR^{a}R^{a}, -NR^{a}C(=O)ONR^{a}R^{a}, -NR^{a}SO₂R^{a}, -N=CR^{a}R^{a}, -SR^{a}, -SOR^{a}, -SO₂R^{a}, -SO₂NR^{a}R^{a}, -NR^{a}SO₂NR^{a}R^{a}, -OSO₂NR^{a}R^{a} und Pseudohalogen; und
R⁵ Wasserstoff, Halogen, C₁₋₃Alkyl, C₂₋₃Alkenyl, C₂₋₃Alkinyl, Halogen-C₁₋₃Alkyl-, -OR^{a} oder Pseudohalogen, und
R⁶ jeweils unabhängig voneinander ein Rest ausgewählt aus der Gruppe bestehend aus Halogen, C₁₋₃Alkyl, -OR^{a} und Pseudohalogen, und
R^{a} jeweils unabhängig voneinander Wasserstoff oder ein Rest ausgewählt aus der Gruppe bestehend aus gegebenenfalls substituiertem C₁₋₈Alkyl, C₂₋₈Alkenyl, C₂₋₈Alkinyl, C₃₋₁₀Cycloalkyl, Aryl, Heterocyclyl und Heteroaryl, wobei der/die Substituent(en) gleich oder verschieden sein können und ausgewählt werden aus der Gruppe bestehend aus Halogen, -NH₂, -OH und Pseudohalogen; und
m 0, 1 oder 2; und
n jeweils unabhängig voneinander 0, 1, oder 2
bedeuten, gegebenenfalls in Form ihrer Tautomeren, ihrer Racemate, ihrer Enantiomere, ihrer Diastereomere und ihrer Gemische, sowie gegebenenfalls ihrer pharmakologisch verträglichen Salze, mit der Maßgabe, dass
2-(2-Methoxy-4-piperazin-1-yl-phenylamino)-4-(3,3-dimethyl-5-oxo-2,3,4,5-benzo[*f*][1,4]oxazepin-6-ylamino-5-trifluormethyl-pyrimidin, 2-(2-Methoxy-4-piperazin-1-yl-phenylamino)-4-((S)-4-oxo-2,3,10,10a-tetrahydro-1*H*,4*H*-9-oxa-3a-aza-benzo[*f*]azulen-5-ylamino-5-trifluormethyl-pyrimidin, 2-[4-(4-Ethyl-piperazin-1-yl)-2-methoxy-phenylamino]-4-((S)-4-oxo-2,3,10,10a-tetrahydro-1*H*,4*H*-9-oxa-3a-aza-benzo[*f*]azulen-5-ylamino-5-trifluormethyl-pyrimidin, 2-[4-(4-Methyl-piperazin-1-yl)-2-methoxy-phenylamino]-4-((S)-4-oxo-2,3,10,10a-tetrahydro-1*H*,4*H*-9-oxa-3a-aza-benzo[*f*]azulen-5-ylamino-5-trifluormethyl-pyrimidin, 3,3-dimethyl-6-{2-[4-(4-methyl-piperazin-1-yl)-phenylamino]-5-trifluormethyl-pyrimidin-4-ylamino}-3,4-dihydro-2*H*-benzo[*f*][1,4]oxazepin-5-on, 6-{2-[4-(4-Ethyl-piperazin-1-yl)-phenylamino]-5-trifluormethyl-pyrimidin-4-ylamino}-3,3-dimethyl-3,4-dihydro-2*H*-benzo[*f*][1,4]oxazepin-5-on 3,3-Dimethyl-6-{2-[4-(4-prop-2-ynyl-piperazin-1-yl)-phenylamino]-5-trifluormethyl-pyrimidin-4-ylamino}-3,4-dihydro-2*H*-benzo[*f*][1,4]oxazepin-5-on, 6-{2-[4-(4-Isopropyl-piperazin-1-yl)-phenylamino]-5-trifluormethyl-pyrimidin-4-ylamino}-3,3-dimethyl-3,4-dihydro-2*H*-benzo[*f*][1,4]oxazepin-5-on, (S)-3-Methyl-6-[2-(4-piperazin-1-yl-phenylamino)-5-trifluormethyl-pyrimidin-4-ylamino]-3,4-dihydro-2*H*-benzo[*f*][1,4]oxazepin-5-on, (S)-3-Methyl-6-{2-[4-(4-methyl-piperazin-1-yl)-phenylamino]-5-trifluormethyl-pyrimidin-4-ylamino}-3,4-dihydro-2*H*-benzo[*f*][1,4]oxazepin-5-on, (S)-6-{2-[4-(4-Isopropyl-piperazin-1-yl)-phenylamino]-5-trifluormethyl-pyrimidin-4-ylamino}-3-methyl-3,4-dihydro-2*H*-benzo[*f*][1,4]oxazepin-5-on, (S)-3-Methyl-6-{2-[4-(4-prop-2-ynyl-piperazin-1-yl)-phenylamino]-5-trifluormethyl-pyrimidin-4-ylamino}-3,4-dihydro-2*H*-benzo[*f*][1,4]oxazepin-5-on (S)-3-Ethyl-6-[2-(4-piperazin-1-yl-phenylamino)-5-trifluormethyl-pyrimidin-4-ylamino]-3,4-dihydro-2*H*-benzo[*f*][1,4]oxazepin-5-on, (S)-6-[2-(4-Piperazin-1-yl-phenylamino)-5-trifluormethyl-pyrimidin-4-ylamino]-3-propyl-3,4-dihydro-2*H*-benzo[*f*][1,4]oxazepin-5-on, (S)-6-{2-[4-(4-Prop-2-ynyl-piperazin-1-yl)-phenylamino]-5-trifluormethyl-pyrimidin-4-ylamino}-1,2,3,11a-tetrahydro-10*H*-benzo[*e*]pyrrolo[1,2-*a*][1,4]diazepin-5,11-dion, (S)-6-{2-[4-(4-Ethyl-piperazin-1-yl)-phenylamino]-5-trifluormethyl-pyrimidin-4-ylamino}-1,2,3,11a-tetrahydro-10*H*-benzo[*e*]pyrrolo[1,2*-a*][1,4]diazepin-5,11-dion, (S)-6-{2-[4-(4-Isobutyl-piperazin-1-yl)-phenylamino]-5-trifluormethyl-pyrimidin-4-ylamino}-1,2,3,11a-tetrahydro-10*H*-benzo[*e*]pyrrolo[1,2-*a*][1,4]diazepin-5,11-dion, (S)-6-{2-[4-(4-Methyl-piperazin-1-yl)-phenylamino]-5-trifluormethyl-pyrimidin-4-ylamino}-1,2,3,11a-tetrahydro-10*H*-benzo[*e*]pyrrolo[1,2*-a*][1,4]diazepin-5,11-dione, 6-{2-[4-(4-Ethyl-piperazin-1-yl)-phenylamino]-5-trifluormethyl-pyrimidin-4-ylamino}-3,3-dimethyl-3,4-dihydro-1*H*-benzo[*e*][1,4]diazepin-2,5-dion, 3,3-Dimethyl-6-{2-[4-(4-methyl-piperazin-1-yl)-phenylamino]-5-trifluormethyl-pyrimidin-4-ylamino}-3,4-dihydro-1*H*-benzo[*e*][1,4]diazepin-2,5-dion, 6-{2-[4-(4-Isopropyl-piperazin-1-yl)-phenylamino]-5-trifluormethyl-pyrimidin-4-ylamino}-3,3-dimethyl-3,4-dihydro-1*H*-benzo[*e*][1,4]diazepin-2,5-dion, 3,3-Dimethyl-6-{2-[4-(4-prop-2-ynyl-piperazin-1-yl)-phenylamino]-5-trifluormethyl-pyrimidin-4-ylamino}-3,4-dihydro-1*H*-benzo[*e*][1,4]diazepin-2,5-dion, (S)-6-{2-[4-(4-Isopropyl-piperazin-1-yl)-phenylamino]-5-trifluormethyl-pyrimidin-4-ylamino}-1,2,3,11a-tetrahydro-10*H*-benzo[*e*]pyrrolo[1,2-*a*][1,4]diazepin-5,11-dion, (S)-6-{2-[4-(4-Isopropyl-piperazin-1-yl)-phenylamino]-5-trifluormethyl-pyrimidin-4-ylamino}-3-methyl-3,4-dihydro-1*H*-benzo[*e*][1,4]diazepin-2,5-dion, (S)-3-Methyl-6-{2-[4-(4-methyl-piperazin-1-yl)-phenylamino]-5-trifluormethyl-pyrimidin-4-ylamino}-3,4-dihydro-1*H*-benzo[*e*][1,4]diazepin-2,5-dion, (S)-3-Methyl-6-{2-[4-(4-prop-2-ynyl-piperazin-1-yl)-phenylamino]-5-trifluormethyl-pyrimidin-4-ylamino}-3,4-dihydro-1*H*-benzo[*e*][1,4]diazepin-2,5-dion, (S)-6-{2-[4-(4-Ethyl-piperazin-1-yl)-phenylamino]-5-trifluormethyl-pyrimidin-4-ylamino}-3-methyl-3,4-dihydro-1*H*-benzo[*e*][1,4]diazepin-2,5-dion und {(S)-2,5-Dioxo-6-[2-(4-piperazin-1-yl-phenylamino)-5-trifluormethyl-pyrimidin-4-ylamino]-2,3,4,5-tetrahydro-1*H*-benzo[*e*][1,4]diazepin-3-yl}-essigsäuremethylester ausgenommen sind.

2. Verbindungen nach Anspruch 1, worin R⁵ Halogen oder -CF₃ bedeutet.

3. Verbindungen nach Anspruch 1 oder 2, worin
X Sauerstoff und
Y -CH₂- bedeuten.

4. Verbindungen nach Anspruch 1 oder 2, worin
X Stickstoff und
Y C=O bedeuten.

5. Verbindungen - oder deren pharmakologisch verträglichen Salze - nach Ansprüchen 1 - 4 als Arzneimittel.

6. Verbindungen - oder deren pharmakologisch verträglichen Salze - nach Ansprüchen 1 - 4 zur Herstellung eines Arzneimittels mit antiproliferativer Wirkung.

7. Verbindungen - oder deren pharmakologisch verträglichen Salze - nach Ansprüchen 1 - 4 zur Herstellung eines Arzneimittels mit antiproliferativer Wirkung mit einem selektiven, kinaseinhibierendem Wirkmechanismus.

8. Verwendung einer Verbindung nach Anspruch 1 - 4 zur Herstellung eines Arzneimittels zur Behandlung und/oder Prävention von Krebs, Infektionen, Entzündungs- und Autoimmunerkrankungen.

9. Pharmazeutische Zubereitung, enthaltend als Wirkstoff eine oder mehrere Verbindungen der allgemeinen Formel (1) gemäß einem der Ansprüche 1 - 4 oder deren pharmakologisch verträglichen Salze gegebenenfalls in Kombination mit üblichen Hilfs- und/oder Trägerstoffen.

10. Pharmazeutische Präparation umfassend eine Verbindungen der allgemeinen Formel (1) gemäß Anspruch 1 - 4, gegebenenfalls in Form ihrer Tautomeren, ihrer Racemate, ihrer Enantiomere, ihrer Diastereomere und ihrer Gemische, sowie gegebenenfalls ihrer pharmakologisch verträglichen Salze, und mindestens eine weitere zytostatische oder zytotoxische Wirksubstanz.

## Claims

1. Compounds of general formula (1) 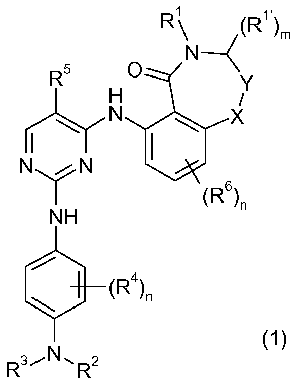 wherein
**X** denotes oxygen or nitrogen, and
**Y** denotes -CH₂- or C=O, and
**R¹** and **R^{1'}** each independently of one another denote hydrogen or an optionally mono- or polysubstituted group selected from among C₁₋₅-alkyl, C₃₋₆-cycloalkyl, aryl, heterocyclyl and heteroaryl, wherein the substituent(s) may be identical or different and are selected from among halogen, -OR^{a}, -C(=O)R^{a}, -C(=O)OR^{a}, -C(=O)NR^{a}R^{a}, -NR^{a}R^{a}, -NR^{a}C(=O)R^{a}, -NR^{a}C(=O)OR^{a}, -NR^{a}C(=O)NR^{a}R^{a}, -NR^{a}SO₂R^{a}, -N=CR^{a}R^{a}, -SR^{a}, -SOR^{a}, -SO₂R^{a}, -SO₂NR^{a}R^{a}, -NR^{a}SO₂NR^{a}R^{a}, -OSO₂NR^{a}R^{a} and pseudohalogen, or optionally the R^{1'} groups on the same carbon atom may form a C₃₋₇-cycloalkyl ring with one another,
or optionally R¹ may together with an R^{1'} form a common saturated or partly saturated 3-6 membered alkyl bridge, which may optionally contain one to two heteroatoms, and
**R²** denotes an optionally mono- or polysubstituted heterocyclyl group, wherein the substituent(s) may be identical or different and are selected from among C₁₋₅-alkyl, C₂₋₅-alkenyl, C₂₋₅-alkynyl, C₃₋₆-cycloalkyl-C₀₋₃-alkyl, and
**R³** denotes hydrogen or C₁₋₃-alkyl, or
**R²** and **R³** together form an optionally mono- or polysubstituted heterocyclyl ring, which may optionally contain one to two further heteroatoms, wherein the substituent(s) may be identical or different and are selected from among C₁₋₅-alkyl, C₂₋₅-alkenyl, C₂₋₅-alkynyl and C₃₋₆-cycloalkyl-C₀₋₃-alkyl, or a suitable substituent selected from among halogen, -OR^{a}, -C(=O)R^{a}, -C(=O)OR^{a}, -C(=O)NR^{a}R^{a}, -NR^{a}R^{a}, -NR^{a}C(=O)R^{a}, -NR^{a}C(=O)OR^{a}, -NR^{a}C(=O)NR^{a}R^{a}, -NR^{a}SO₂R^{a}, -N=CR^{a}R^{a}, -SR^{a}, -SOR^{a}, -SO₂R^{a}, -SO₂NR^{a}R^{a}, -NR^{a}SO₂NR^{a}R^{a}, -OSO₂NR^{a}R^{a} and pseudohalogen, and
**R⁴** each independently of one another denote a group selected from among halogen, NO₂, -OR^{a}, -C(=O)R^{a}, -C(=O)OR^{a}, -C(=O)NR^{a}R^{a}, -NR^{a}R^{a}, -NR^{a}COR^{a}, -NR^{a}C(=O)OR^{a}, -NR^{a}C(=O)NR^{a}R^{a}, -NR^{a}C(=O)ONR^{a}R^{a}, -NR^{a}SO₂R^{a}, -N=CR^{a}R^{a}, -SR^{a}, -SOR^{a}, -SO₂R^{a}, -SO₂NR^{a}R^{a}, -NR^{a}SO₂NR^{a}R^{a}, -OSO₂NR^{a}R^{a} and pseudohalogen; and
**R⁵** denotes hydrogen, halogen, C₁₋₃-alkyl, C₂₋₃-alkenyl, C₂₋₃-alkynyl, halogen-C₁₋₃-alkyl-, -OR^{a} or pseudohalogen, and
**R⁶** each independently of one another denote a group selected from among halogen, C₁₋₃₋alkyl, -OR^{a} and pseudohalogen, and
**R^{a}** each independently of one another denote hydrogen or a group selected from among optionally substituted C₁₋₈₋alkyl, C₂₋₈-alkenyl, C₂₋₈₋alkynyl, C₃₋₁₀₋cycloalkyl, aryl, heterocyclyl and heteroaryl, while the substituent(s) may be identical or different and are selected from among halogen, -NH₂, -OH and pseudohalogen; and
**m** denotes 0, 1 or 2; and
**n** each independently of one another denote 0, 1, or 2,
optionally in the form of the tautomers, the racemates, the enantiomers, the diastereomers and the mixtures thereof, as well as optionally the pharmacologically acceptable salts thereof, with the proviso that
2-(2-methoxy-4-piperazin-1-yl-phenylamino)-4-(3,3-dimethyl-5-oxo-2,3,4,5-benzo[*f*][1,4]oxazepin-6-ylamino-5-trifluoromethyl-pyrimidine, 2-(2-methoxy-4-piperazin-1-yl-phenylamino)-4-((S)-4-oxo-2,3,10,10a-tetrahydro-1*H*,4*H*-9-oxa-3a-aza-benzo[*f*]azulen-5-ylamino-5-trifluoromethyl-pyrimidine, 2-[4-(4-ethyl-piperazin-1-yl)-2-methoxy-phenylamino]-4-((S)-4-oxo-2,3,10,10a-tetrahydro-1*H*,4*H*-9-oxa-3a-aza-benzo[*f*]azulen-5-ylamino-5-trifluoromethyl-pyrimidine, 2-[4-(4-methyl-piperazin-1-yl)-2-methoxy-phenylamino]-4-((S)-4-oxo-2,3,10,10a-tetrahydro-1*H*,4*H*-9-oxa-3a-aza-benzo[*f*]azulen-5-ylamino-5-trifluoromethyl-pyrimidine, 3,3-dimethyl-6-{2-[4-(4-methyl-piperazin-1-yl)-phenylamino]-5-trifluoromethyl-pyrimidin-4-ylamino}-3,4-dihydro-2*H*-benzo[*f*][1,4]oxazepin-5-one, 6-{2-[4-(4-ethyl-piperazin-1-yl)-phenylamino]-5-trifluoromethyl-pyrimidin-4-ylamino}-3,3-dimethyl-3,4-dihydro-2*H*-benzo[*f*][1,4]oxazepin-5-one, 3,3-dimethyl-6-{2-[4-(4-prop-2-ynyl-piperazin-1-yl)-phenylamino]-5-trifluoromethyl-pyrimidin-4-ylamino}-3,4-dihydro-2*H*-benzo[*f*][1,4]oxazepin-5-one, 6-{2-[4-(4-isopropyl-piperazin-1-yl)-phenylamino]-5-trifluoromethyl-pyrimidin-4-ylamino}-3,3-dimethyl-3,4-dihydro-2*H*-benzo[*f*][1,4]oxazepin-5-one, (S)-3-methyl-6-[2-(4-piperazin-1-yl-phenylamino)-5-trifluoromethyl-pyrimidin-4-ylamino]-3,4-dihydro-2*H*-benzo[*f*][1,4]oxazepin-5-one, (S)-3-methyl-6-{2-[4-(4-methyl-piperazin-1-yl)-phenylamino]-5-trifluoromethyl-pyrimidin-4-ylamino}-3,4-dihydro-2*H*-benzo[*f*][1,4]oxazepin-5-one, (S)-6-{2-[4-(4-isopropyl-piperazin-1-yl)-phenylamino]-5-trifluoromethyl-pyrimidin-4-ylamino}-3-methyl-3,4-dihydro-2*H*-benzo[*f*][1,4]oxazepin-5-one, (S)-3-methyl-6-{2-[4-(4-prop-2-ynyl-piperazin-1-yl)-phenylamino]-5-trifluoromethyl-pyrimidin-4-ylamino}-3,4-dihydro-2*H*-benzo[*f*][1,4]oxazepin-5-one, (S)-3-ethyl-6-[2-(4-piperazin-1-yl-phenylamino)-5-trifluoromethyl-pyrimidin-4-ylamino]-3,4-dihydro-2*H*-benzo[*f*][1,4]oxazepin-5-one, (S)-6-[2-(4-piperazin-1-yl-phenylamino)-5-trifluoromethyl-pyrimidin-4-ylamino]-3-propyl-3,4-dihydro-2*H*-benzo[*f*][1,4]oxazepin-5-one, (S)-6-{2-[4-(4-prop-2-ynyl-piperazin-1-yl)-phenylamino]-5-trifluoromethyl-pyrimidin-4-ylamino}-1,2,3,11a-tetrahydro-10*H*-benzo[*e*]pyrrolo[1,2-*a*][1,4]diazepine-5,11-dione, (S)-6-{2-[4-(4-ethyl-piperazin-1-yl)-phenylamino]-5-trifluoromethyl-pyrimidin-4-ylamino}-1,2,3,11a-tetrahydro-10*H*-benzo[*e*]pyrrolo[1,2-*a*][1,4]diazepine-5,11-dione, (S)-6-{2-[4-(4-isobutyl-piperazin-1-yl)-phenylamino]-5-trifluoromethyl-pyrimidin-4-ylamino}-1,2,3,11a-tetrahydro-10*H*-benzo[*e*]pyrrolo[1,2-*a*][1,4]diazepine-5,11-dione, (S)-6-{2-[4-(4-methyl-piperazin-1-yl)-phenylamino]-5-trifluoromethyl-pyrimidin-4-ylamino}-1,2,3,11a-tetrahydro-10*H*-benzo[*e*]pyrrolo[1,2-*a*][1,4]diazepine-5,11-dione, 6-{2-[4-(4-ethyl-piperazin-1-yl)-phenylamino]-5-trifluoromethyl-pyrimidin-4-ylamino}-3,3-dimethyl-3,4-dihydro-1*H-*benzo[*e*][1,4]diazepine-2,5-dione, 3,3-dimethyl-6-{2-[4-(4-methyl-piperazin-1-yl)-phenylamino]-5-trifluoromethyl-pyrimidin-4-ylamino}-3,4-dihydro-1*H*-benzo[*e*][1,4]diazepine-2,5-dione, 6-{2-[4-(4-isopropyl-piperazin-1-yl)-phenylamino]-5-trifluoromethyl-pyrimidin-4-ylamino}-3,3-dimethyl-3,4-dihydro-1*H*-benzo[*e*][1,4]diazepine-2,5-dione, 3,3-dimethyl-6-{2-[4-(4-prop-2-ynyl-piperazin-1-yl)-phenylamino]-5-trifluoromethyl-pyrimidin-4-ylamino}-3,4-dihydro-1*H*-benzo[*e*][1,4]diazepine-2,5-dione, (S)-6-{2-[4-(4-isopropyl-piperazin-1-yl)-phenylamino]-5-trifluoromethyl-pyrimidin-4-ylamino}-1,2,3,11a-tetrahydro-10*H*-benzo[*e*]pyrrolo[1,2-*a*][1,4]diazepine-5,11-dione, (S)-6-{2-[4-(4-isopropyl-piperazin-1-yl)-phenylamino]-5-trifluoromethyl-pyrimidin-4-ylamino}-3-methyl-3,4-dihydro-1*H*-benzo[*e*][1,4]diazepine-2,5-dione, (S)-3-methyl-6-{2-[4-(4-methyl-piperazin-1-yl)-phenylamino]-5-trifluoromethyl-pyrimidin-4-ylamino}-3,4-dihydro-1*H*-benzo[*e*][1,4]diazepine-2,5-dione, (S)-3-methyl-6-{2-[4-(4-prop-2-ynyl-piperazin-1-yl)-phenylamino]-5-trifluoromethyl-pyrimidin-4-ylamino}-3,4-dihydro-1*H*-benzo[*e*][1,4]diazepine-2,5-dione, (S)-6-{2-[4-(4-ethyl-piperazin-1-yl)-phenylamino]-5-trifluoromethyl-pyrimidin-4-ylamino}-3-methyl-3,4-dihydro-1*H*-benzo[*e*][1,4]diazepine-2,5-dione and methyl {(S)-2,5-dioxo-6-[2-(4-piperazin-1-yl-phenylamino)-5-trifluoromethyl-pyrimidin-4-ylamino]-2,3,4,5-tetrahydro-1*H*-benzo[*e*][1,4]diazepin-3-yl}-acetate are excluded.

2. Compounds according to claim 1, wherein **R⁵** denotes halogen or -CF₃.

3. Compounds according to claim 1 or 2, wherein
**X** denotes oxygen and
**Y** denotes -CH₂-.

4. Compounds according to claim 1 or 2, wherein
**X** denotes nitrogen and
**Y** denotes C=O.

5. Compounds - or the pharmacologically acceptable salts thereof - according to Claims 1 - 4 as medicaments.

6. Compounds - or the pharmacologically acceptable salts thereof - according to Claims 1 - 4 for preparing a medicament with an antiproliferative action.

7. Compounds - or the pharmacologically acceptable salts thereof - according to Claims 1 - 4 for preparing a medicament with an antiproliferative action with a selective, kinase-inhibiting mechanism of activity.

8. Use of a compound according to claim 1 - 4 for preparing a medicament for the treatment and/or prevention of cancer, infections, inflammatory and autoimmune diseases.

9. Pharmaceutical preparation, containing as active substance one or more compounds of general formula (1) according to one of claims 1 - 4 or the pharmacologically acceptable salts thereof optionally in combination with conventional excipients and/or carriers.

10. Pharmaceutical preparation, comprising a compound of general formula (1) according to claim 1 - 4, optionally in the form of the tautomers, the racemates, the enantiomers, the diastereomers and the mixtures thereof, as well as optionally the pharmacologically acceptable salts thereof, and at least one further cytostatic or cytotoxic active substance.

## Revendications

1. Composés de formule générale (1) 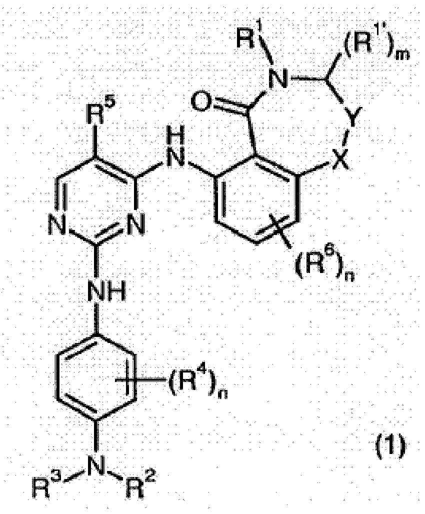 dans laquelle
X est un atome d'oxygène ou d'azote, et
Y est -CH₂- ou C=O, et
R¹ et R^{1'} sont respectivement, indépendamment l'un de l'autre, un atome d'hydrogène ou un radical éventuellement mono- ou multi-substitué choisi dans le groupe constitué des groupes alkyle en C₁ à C₅, cycloalkyle en C₃ à C₆, aryle, hétérocyclyle et hétéroaryle, le/les substituants(s) pouvant être identique(s) ou différent(s) et être choisi(s) dans le groupe constitué d'un atome d'halogène, - OR^{a}, -C(=O)R^{a}, -C(=O)OR^{a}, -C(=O)NR^{a}R^{a}, -NR^{a}R^{a}, -NR^{a}C(=O)R^{a}, - NR^{a}C(=O)OR^{a}, -NR^{a}C(=O)NR^{a}R^{a}, -NR^{a}SO₂R^{a}, -N=CR^{a}R^{a}, -SR^{a}, -SOR^{a}, -SO₂R^{a}, - SO₂NR^{a}R^{a}, -NR^{a}SO₂NR^{a}R^{a}, -OSO₂NR^{a}R^{a} et un pseudo-halogène ou éventuellement, les R^{1'} se trouvant sur le même atome de C peuvent former ensemble un cycle cycloalkyle en C₃ à C₇,
ou éventuellement, R¹ peut être relié à R^{1'} formant conjointement un pont alkyle de 3 à 6 chaînons saturé ou partiellement insaturé qui peut contenir éventuellement un à deux hétéroatomes, et
R² est un radical hétérocyclyle éventuellement mono- ou multi-substitué, le/les substituant(s) pouvant être identiques ou différents et être choisis dans le groupe constitué des groupes alkyle en C₁ à C₅, alcényle en C₂ à C₅, alcinyle en C₂ à C₅, cycloalkyle en C₃ à C₆-alkyle en C₀ à C₃, et
R³ est un atome d'hydrogène ou un groupe alkyle en C₁ à C₃, ou
R² et R³ forment conjointement un cycle hétérocyclyle éventuellement mono- ou multi-substitué qui peut contenir éventuellement un à deux autres hétéroatomes, le/les substituant(s) pouvant être identiques ou différents et être choisis dans le groupe constitué des groupes alkyle en C₁ à C₅, alcényle en C₂ à C₅, alcinyle en C₂ à C₅ et cycloalkyle en C₃ à C₆-alkyle en C₀ à C₃, ou un substituant approprié choisi dans le groupe constitué d'un atome d'halogène, -OR^{a}, -C(=O)R^{a}, - C(=O)OR^{a}, -C(=O)NR^{a}R^{a}, -NR^{a}R^{a}, -NR^{a}C(=O)R^{a}, -NR^{a}C(=O)OR^{a}, - NR^{a}C(=O)NR^{a}R^{a}, -NR^{a}SO₂R^{a}, -N=CR^{a}R^{a}, -SR^{a}, -SOR^{a}, -SO₂R^{a}, -SO₂NR^{a}R^{a}, - NR^{a}SO₂NR^{a}R^{a}, -OSO₂NR^{a}R^{a} et un pseudo-halogène, et
les R⁴ sont respectivement, indépendamment les uns des autres, un radical choisi dans le groupe constitué d'un atome d'halogène, NO₂, -OR^{a}, -C(=O)R^{a}, - C(=O)OR^{a}, -C(=O)NR^{a}R^{a}, -NR^{a}R^{a}, -NR^{a}COR^{a}, -NR^{a}C(=O)OR^{a}, -NR^{a}C(=O)NR^{a}R^{a}, -NR^{a}C(=O)ONR^{a}R^{a}, -NR^{a}SO₂R^{a}, -N=CR^{a}R^{a}, -SR^{a}, -SOR^{a}, -SO₂R^{a}, -SO₂NR^{a}R^{a}, - NR^{a}SO₂NR^{a}R^{a}, -OSO₂NR^{a}R^{a} et un pseudo-halogène ; et
R⁵ est un atome d'hydrogène, d'halogène, un groupe alkyle en C₁ à C₃, alcényle en C₂ à C₃, alcinyle en C₂ à C₃, halogéno-alkyle en C₁ à C₃, -OR^{a} ou un pseudo-halogène, et
les R⁶ sont respectivement, indépendamment les uns des autres, un radical choisi dans le groupe constitué d'un atome d'halogène, un groupe alkyle en C₁ à C₃, -OR^{a} et un pseudo-halogène, et
les R^{a} sont respectivement, indépendamment les uns des autres, un atome d'hydrogène ou un radical choisi dans le groupe constitué d'un groupe alkyle en C₁ à C₈ éventuellement substitué, alcényle en C₂ à C₈,
alcinyle en C₂ à C₈, cycloalkyle en C₃ à C₁₀, aryle, hétérocyclyle et hétéroaryle, le/les substituant(s) pouvant être identique(s) ou différent(s) et être choisis dans le groupe constitué d'un atome d'halogène, -NH₂, -OH et un pseudo-halogène ; et
m est 0, 1 ou 2 ; et
les n sont respectivement, indépendamment les uns des autres, 0, 1 ou 2,
éventuellement sous la forme de leurs tautomères, leurs racémates, leurs énantiomères, leurs diastéréomères et leurs mélanges, et éventuellement, leurs sels pharmacologiquement acceptables, à condition que
la 2-(2-méthoxy-4-pipérazin-1-yl-phénylamino)-4-(3,3-diméthyl-5-oxo,2,3,4,5-benzo[*f*][1,4]oxazépin-6-ylamino-5-trifluorométhyl-pyrimidine,
la 2-(2-méthoxy-4-pipérazin-1-yl-phénylamino)-4-((S)-4-oxo,2,3,10,10a-tétrahydro-1*H*,4*H*-9-oxa-3a-aza-benzo[*f*]azulén-5-ylamino-5-trifluorométhyl-pyrimidine,
la 2-[4-(4-éthyl-pipérazin-1-yl)-2-méthoxy-phénylamino]-4-((S)-4-oxo-2,3,10,10a-tétrahydro-1*H*,4*H*-9-oxa-3a-aza-benzo[*f*]azulén-5-ylamino-5-trifluorométhyl-pyrimidine,
la 2-[4-(4-méthyl-pipérazin-1-yl)-2-méthoxy-phénylamino]-4-((S)-4-oxo-2,3,10,10a-tétrahydro-1*H*,4*H*-9-oxa-3a-aza-benzo[*f*]azulén-5-ylamino-5-trifluorométhyl-pyrimidine,
la 3,3-diméthyl-6-{2-[4-(4-méthyl-pipérazin-1-yl)-phénylamino]-5-trifluorométhyl-pyrimidin-4-ylamino}-3,4-dihydro-2*H*-benzo[*f*][1,4]oxazépin-5-one,
la 6-{2-[4-(4-éthyl-pipérazin-1-yl)-phénylamino]-5-trifluorométhyl-pyrimidin-4-ylamino}-3,3-diméthyl-3,4-dihydro-2*H*-benzo[*f*][1,4]oxazépin-5-one,
la 3,3-diméthyl-6-{2-[4-(4-prop-2-ynyl-pipérazin-1-yl)-phénylamino]-5-trifluorométhyl-pyrimidin-4-ylamino}-3,4-dihydro-2*H*-benzo[*f*][1,4]oxazépin-5-one,
la 6-{2-[4-(4-isopropyl-pipérazin-1-yl)-phénylamino]-5-trifluorométhyl-pyrimidin-4-ylamino}-3,3-diméthyl-3,4-dihydro-2*H*-benzo[*f*][1,4]oxazépin-5-one,
la (S)-3-méthyl-6-[2-(4-pipérazin-1-yl-phénylamino)-5-trifluorométhyl-pyrimidin-4-ylamino]-3,4-dihydro-2*H*-benzo[*f*][1,4]oxazépin-5-one,
la (S)-3-méthyl-6-{2-[4-(4-méthyl-pipérazin-1-yl)-phénylamino]-5-trifluorométhyl-pyrimidin-4-ylamino}-3,4-dihydro-2*H*-benzo[*f*][1,4]oxazépin-5-one,
la (S)-6-{2-[4-(4-isopropyl-pipérazin-1-yl)-phénylamino]-5-trifluorométhyl-pyrimidin-4-ylamino}-3-méthyl-3,4-dihydro-2*H*-benzo[*f*][1,4]oxazépin-5-one,
la (S)-3-méthyl-6-{2-[4-(4-prop-2-ynyl-pipérazin-1-yl)-phénylamino]-5-trifluorométhyl-pyrimidin-4-ylamino}-3,4-dihydro-2*H*-benzo[*f*][1,4]oxazépin-5-one,
la (S)-3-éthyl-6-[2-(4-pipérazin-1-yl-phénylamino)-5-trifluorométhyl-pyrimidin-4-ylamino]-3,4-dihydro-2*H*-benzo[*f*][1,4]oxazépin-5-one,
la (S)-6-[2-(4-pipérazin-1-yl-phénylamino)-5-trifluoro-méthyl-pyrimidin-4-ylamino]-3-propyl-3,4-dihydro-2*H*-benzo[*f*][1,4]oxazépin-5-one,
la (S)-6-{2-[4-(4-prop-2-ynyl-pipérazin-1-yl)-phénylamino]-5-trifluorométhyl-pyrimidin-4-ylamino}-1,2,3,11a-tétrahydro-10*H*-benzo[*e*]pyrrolo[1,2-*a* ][1,4]diazépin-5,11-dione,
la (S)-6-{2-[4-(4-éthyl-pipérazin-1-yl)-phénylamino]-5-trifluoro-méthyl-pyrimidin-4-ylamino}-1,2,3,11a-tétrahydro-10*H*-benzo[*e*]pyrrolo[1,2-*a* ][1,4]diazépin-5,11-dione,
la (S)-6-{2-[4-(4-isobutyl-pipérazin-1-yl)-phénylamino]-5-trifluoro-méthyl-pyrimidin-4-ylamino}-1,2,3,11a-tétrahydro-10*H*-benzo[*e*]pyrrolo[1,2-*a* ][1,4]diazépin-5,11-dione,
la (S)-6-{2-[4-(4-méthyl-pipérazin-1-yl)-phénylamino]-5-trifluorométhyl-pyrimidin-4-ylamino}-1,2,3,11a-tétrahydro-10*H*-benzo[*e*]pyrrolo[1,2-*a* ][1,4]diazépin-5,11-dione,
la 6-{2-[4-(4-éthyl-pipérazin-1-yl)-phénylamino]-5-trifluorométhyl-pyrimidin-4-ylamino}-3,3-diméthyl-3,4-dihydro-1*H*-benzo[*e*][1,4]diazépin-2,5-dione,
la 3,3-diméthyl-6-{2-[4-(4-méthyl-pipérazin-1-yl)-phénylamino]-5-trifluorométhyl-pyrimidin-4-ylamino}-3,4-dihydro-1*H*-benzo[*e*][1,4]diazépin-2,5-dione,
la 6-{2-[4-(4-isopropyl-pipérazin-1-yl)-phénylamino]-5-trifluorométhyl-pyrimidin-4-ylamino}-3,3-diméthyl-3,4-dihydro-1*H*-benzo[*e*][1,4]diazépin-2,5-dione,
la 3,3-diméthyl-6-{2-[4-(4-prop-2-ynyl-pipérazin-1-yl)-phénylamino]-5-trifluorométhyl-pyrimidin-4-ylamino}-3,4-dihydro-1*H*-benzo[*e*][1,4]diazépin-2,5-dione,
la (S)-6-{2-[4-(4-isopropyl-pipérazin-1-yl)-phénylamino]-5-trifluorométhyl-pyrimidin-4-ylamino}-1,2,3,11a-tétrahydro-10*H*-benzo[*e*]pyrrolo[1,2-*a* ][1,4]diazépin-5,11-dione,
la (S)-6-{2-[4-(4-isopropyl-pipérazin-1-yl)-phénylamino]-5-trifluorométhyl-pyrimidin-4-ylamino}-3-méthyl-3,4-dihydro-1*H*-benzo[*e*][1,4]diazépin-2,5-dione,
la (S)-3-méthyl-6-{2-[4-(4-méthyl-pipérazin-1-yl)-phénylamino]-5-trifluorométhyl-pyrimidin-4-ylamino}-3,4-dihydro-1*H*-benzo[*e*][1,4]diazépin-2,5-dione,
la (S)-3-méthyl-6-{2-[4-(4-prop-2-ynyl-pipérazin-1-yl)-phénylamino]-5-trifluorométhyl-pyrimidin-4-ylamino}-3,4-dihydro-1*H*-benzo[*e*][1,4]diazépin-2,5-dione,
la (S)-6-{2-[4-(4-éthyl-pipérazin-1-yl)-phénylamino]-5-trifluorométhyl-pyrimidin-4-ylamino}-3-méthyl-3,4-dihydro-1*H*-benzo[*e*][1,4]diazépin-2,5-dione et
le méthylester de l'acide {(S)-2,5-dioxo-6-[2-(4-pipérazin-1-yl-phénylamino)-5-trifluorométhyl-pyrimidin-4-ylamino]-2,3,4,5-tétrahydro-1*H*-benzo[*e*][1,4]diazépin-3-yl}-acétique soient exclus.

2. Composés selon la revendication 1, dans lesquels R⁵ est un atome d'halogène ou -CF₃.

3. Composés selon la revendication 1 ou 2, dans lesquels
X est un atome d'oxygène et
Y est -CH₂-.

4. Composés selon la revendication 1 ou 2, dans lesquels
X est un atome d'azote et
Y est C=O.

5. Composés ou leurs sels pharmacologiquement acceptables, selon les revendications 1 à 4, comme médicaments.

6. Composés, ou leurs sels pharmacologiquement acceptables, selon les revendications 1 à 4, pour la production d'un médicament à effet antiprolifératif.

7. Composés, ou leurs sels pharmacologiquement acceptables, selon les revendications 1 à 4, pour la production d'un médicament à effet antiprolifératif comportant un mécanisme d'action sélectif, inhibant les kinases.

8. Utilisation d'un composé selon la revendication 1 à 4, pour la production d'un médicament pour le traitement et/ou la prévention du cancer, des infections, des maladies inflammatoires et auto-immunes.

9. Préparation pharmaceutique contenant comme substance active, un ou plusieurs composés de formule générale (1) selon l'une des revendications 1 à 4 ou leurs sels pharmacologiquement acceptables éventuellement en combinaison avec des adjuvants et/ou des véhicules habituels.

10. Préparation pharmaceutique comprenant un composé de formule générale (1) selon la revendication 1 à 4, éventuellement sous la forme de leurs tautomères, leurs racémates, leurs énantiomères, leurs diastéréomères et leurs mélanges et éventuellement, leurs sels pharmacologiquement acceptables et au moins une autre substance active cytostatique ou cytotoxique.


### SEQUENCE LISTING

<110> Boehringer Ingelheim International GmbH
<120> Neue Verbindungen
<130> Case 12-0252
<160> 3
<170> PatentIn version 3.1
<210> 1
   <211> 13
   <212> PRT
   <213> Artificial sequence
<220>
   <223> Synthetic Peptide
<400> 1
   Glu Pro Leu Glu Arg Arg Leu Ser Leu Val Pro Asp Ser
   1               5                   10
<210> 2
   <211> 13
   <212> PRT
   <213> Artificial sequence
<220>
   <223> Synthetic Peptide
<400> 2
   Glu Pro Leu Glu Arg Arg Leu Ser Leu Val Pro Lys Met
   1               5                   10
<210> 3
   <211> 28
   <212> PRT
   <213> Artificial sequence
<220>
   <223> Synthetic peptide
<400> 3
   Leu Arg Arg Trp Ser Leu Gly Leu Arg Arg Trp Ser Leu Gly Leu Arg
   1               5                   10                  15
   Arg Trp Ser Leu Gly Leu Arg Arg Trp Ser Leu Gly
               20                  25